# EUROPEAN PATENT SPECIFICATION

(11) **EP 3 833 335 B1**
(45) Date of publication and mention of the grant of the patent: **31.08.2022**
(21) Application number: 20764155.6
(22) Date of filing: 30.07.2020
(51) Int. Cl.: A61P 3/06, A61K 9/24, A61K 31/505, A61K 31/216, A61K 9/20

(54) **PHARMACEUTICAL COMPOSITION COMPRISING HMG-COA REDUCTASE INHIBITORS AND FENOFIBRATE**
PHARMAZEUTISCHE ZUSAMMENSETZUNG MIT HMG-COA-REDUKTASE-HEMMERN UND FENOFIBRAT
COMPOSITION PHARMACEUTIQUE COMPRENANT DES INHIBITEURS DE RÉDUCTASE HMG-COA ET DU FÉNOFIBRATE

(30) Priority: 31.07.2019 IN 201921030870; 02.10.2019 EP 19201186
(43) Date of publication of application: 16.06.2021
(73) Proprietor: Intas Pharmaceuticals Ltd., Ahmedabad - 380054, Gujarat (IN)
(72) Inventor: RAHANGDALE, Hitendra Netlal, Tal.: Sanand Ahmedabad, Gujarat 382210 (IN); PRAJAPATI, Narendra Bhagwatbhai, Tal.: Sanand Ahmedabad, Gujarat 382210 (IN); DHAVALE, Satyavan Shivajirao, Tal.: Sanand Ahmedabad, Gujarat 382210 (IN); NAIDU, Venkataramana, Tal.: Sanand Ahmedabad, Gujarat 382210 (IN)
(74) Representative: Ter Meer Steinmeister & Partner
(86) International application number: PCT/IB2020/057208
(87) International publication number: WO 2021/019493

(56) References cited:
- WO-A2-2008/075320
- Lokesh Kumar ET AL: "COMBINATIONAL THERAPY OF ROSUVASTATIN CALCIUM AND FENOFIBRATE AS BILAYER TABLET: A POTENTIAL APPROACH TO CONTROL HYPOLIPIDAEMIA", International Journal of Pharmaceutical Sciences and Research, 1 January 2016 (2016-01-01), XP055674904, DOI: 10.13040/IJPSR.0975-8232.7(1).413-28 Retrieved from the Internet: URL:http://ijpsr.com/wp-content/uploads/20 16/01/53-Vol.-7-Issue-1-Jan-2016-IJPSR-RA- 6127-Paper-53.pdf

## Description

### FIELD OF THE INVENTION

The present invention provides a multilayer, pharmaceutical composition comprising a fixed dose combination of rosuvastatin or a pharmaceutically acceptable salt thereof and fenofibrate or a pharmaceutically acceptable salt thereof, wherein the fenofibrate or the pharmaceutically acceptable salt thereof and the rosuvastatin or the pharmaceutically acceptable salt thereof are present in separate layers, namely a fenofibrate layer and a rosuvastatin layer, wherein fenofibrate and rosuvastatin are immediately released from the fenofibrate layer and the rosuvastatin layer, respectively, and wherein the fenofibrate layer comprises micronized fenofibrate. Further, the present invention provides a process for the preparation of the said composition.

### BACKGROUND OF THE INVENTION

Rosuvastatin is a selective and competitive inhibitor of HMG-CoA reductase inhibitor and has a lipid lowering activity. The chemical name of rosuvastatin is bis[(E)-7-[4(4-fluorophenyl)-6-isopropyl-2-[methyl(methylsulfonyl)amino]-pyrimidin-5-yl](3R,5S)-3,5dihydroxyhept-6-enoic acid]. Rosuvastatin corresponds to the compound with the following structure (Formula I): 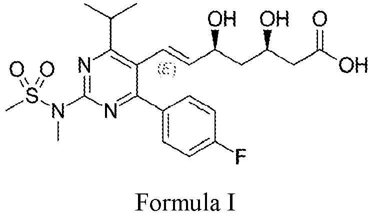 Rosuvastatin is, for instance, known from US RE37 314 E and EP 0 521 471 B1. Both prior art documents disclose that rosuvastatin is especially effective as HMG-CoA reductase inhibitor. Moreover, rosuvastatin calcium is marketed as a single drug formulation with the name CRESTOR^{®} in the form of 5 mg; 10 mg; 20 mg and 40 mg tablets. CRESTOR^{®} has two approved therapeutic indications, namely, the treatment of hypercholesterolemia in adults, adolescents and children aged 6 years or older, and the prevention of cardiovascular events.

The treatment of hypercholesterolemia is a long-term treatment which may encompass a first initial oral treatment phase followed by a second dose adjustment phase. Particularly, the recommended start dose is 5 or 10 mg once daily followed by a dose adjustment switch to 20 or 40 mg, which can be made, if necessary, after 4 weeks of treatment. For the prevention of cardiovascular events, the used dose is 20 mg.

Following oral administration, rosuvastatin undergoes limited metabolism. *In vitro* metabolism studies using human hepatocytes indicate that rosuvastatin is a poor substrate for cytochrome P450-based metabolism.

EP 2 306 982 B1 aims at improving the dissolution profile of CRESTOR^{®}. To this end, it discloses a pharmaceutical composition in which rosuvastatin calcium is provided together with an alkali metal carbonate, bicarbonate or a mixture thereof. The molar ratio of these components is 1 : 0.43-1.75 (rosuvastatin calcium : alkali metal carbonate, bicarbonate or a mixture thereof). This composition is reported to have a beneficial dissolution profile of rosuvastatin calcium in a 0.1 N HCl medium provided that the anhydrous form of sodium carbonate is present in a range of 0.5 % to 2 % by weight of the pharmaceutical composition. The pharmaceutical composition is preferably an oral dosage form such as a tablet or another oral dosage form such as a capsule, a pellet, a minitablet or the like.

Fenofibrate is an activator of peroxisome proliferator-activated receptor alpha (PPARα). Through activation of PPARα, fenofibrate increases the lipolysis and elimination of atherogenic triglyceride-rich particles from plasma by activating lipoprotein lipase and reducing production of apoprotein CIII. Activation of PPARα also induces an increase in the synthesis of apoproteins AI and AII. Chemically speaking, fenofibrate is 2-[4-(4-chlorobenzoyl) phenoxy]-2-methyl-propanoic acid 1-methylethyl ester and has the following structure (Formula II):

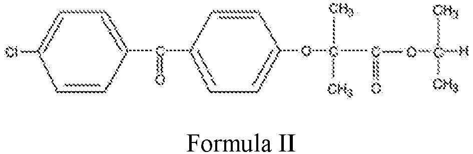

Fenofibrate is disclosed in GB 1 415 295 A. Additionally, fenofibrate is commercially available and marketed in a single drug formulation in different pharmaceutical forms and dosages, e.g. as TRICOR^{®} in the form of 54 mg and 160 mg tablets; as either SUPRALIP^{®} in the UK or as SECALIP SUPRA^{®} in Spain in the form of 160 mg tablets; and as LIPANTHYL^{®} in the form of 200 mg capsules.

In the case of SUPRALIP^{®}/ SECALIP SUPRA^{®} 160 mg tablets, it is indicated for the treatment of severe hypertriglyceridaemia with or without low HDL cholesterol, for mixed hyperlipidaemia when a statin is not tolerated, as well as for the treatment of mixed hyperlipidaemia in patients at high cardiovascular risk. The recommended dose for said indications is one tablet containing 160 mg of fenofibrate taken once daily. Following oral administration, fenofibrate is rapidly hydrolyzed to esterases to the active metabolite, fenofibric acid. Fenofibric acid is primarily conjugated with glucuronic acid and then excreted in the urine.

US 6 277 405 B1 pertains to a method for preparing a composition with an allegedly improved fenofibrate dissolution when compared to some of the commercially available products. The method comprises the steps of: (a) preparing a fenofibrate suspension in micronized form with a particle size below 20 µm, in a solution of hydrophilic polymer and, optionally surfactant, (b) applying the suspension from step (a) to an inert hydrosoluble carrier. The disclosed composition purportedly exhibits a fenofibrate dissolution of at least 75% in 30 minutes, as measured using the rotating blade method at 75 rpm according to Ph. Eur. in a dissolution medium constituted by water with 2% by weight polysorbate 80 or in a dissolution medium constituted by water with 0.025M of sodium lauryl sulphate.

US 2011/ 0 311 625 A1 teaches that the solubility of an active pharmaceutical ingredient influences the bioavailability of a drug. Further, it teaches that fenofibrate is a poorly soluble drug. Due to its poor hydrosolubility, fenofibrate poses the problem of low dissolution. It is poorly absorbed in the digestive tract and consequently its bioavailability is considered incomplete and irregular. The poor dissolution properties are claimed to be mitigated by providing the fenofibrate in a specific form, namely in a tablet comprising a core and a layer surrounding the core, wherein the layer surrounding the core comprises the fenofibrate, a hydrophilic polymer and a hydrophilic carrier.

EP 0 330 532 B1 suggests a new therapeutic composition, which contains fenofibrate that has been co-micronized with a solid surfactant. The new composition is claimed to have an improved fenofibrate dissolution and, thus, an increased bioavailability. The daily dose of medicament may be reduced from 300 mg to 200 mg and from 100 mg to 67 mg without having to make any compromises with respect to the efficiency of the therapy.

Instead of single drug formulations, some prior art references suggest using fenofibrate and a HMG-CoA inhibitor in combination. This should be the most effective means of cholesterol and lipid management. In fact, treatment with fenofibrate is often prescribed together with a statin as clinicians seem to prefer the use of e.g. fenofibrate due to its triglyceride-lowering and HDL-C increasing effects while a HMG-CoA inhibitor is used for its positive effects on lowering LDL-C and raising HDL-C and/or related disease. Fixed-dose combinations including a fibrate and a statin as active pharmaceutical ingredients are known from the documents below.

EP 1 185 274 B1, for instance, discloses a combination of the HMG-CoA reductase inhibitor rosuvastatin with an inducer, inhibitor or substrate of P450 isoenzyme 3A4 for simultaneous, separate or sequential use in therapy.

WO 2005/ 034 908 A2 discloses pharmaceutical compositions comprising a combination of a fibrate and a statin and a common vehicle. The vehicle is selected from the group consisting of a hydrophobic, a hydrophilic and a water-miscible vehicle. At least 80% w/w, but preferably 99.9% w/w of the total amount of the fibrate and the statin (the active substances) is dissolved in the common vehicle. Realizing such a solution is, however, technically demanding. The fenofibrate and the statin have to be dissolved in a mixture of PEG 6000 and Poloxamer 188 (70:30) or in a glyceryl monostearate at 70°C and the solution has to be subsequently sprayed on lactose in a fluid bed. Moreover, the release characteristics of fenofibrate from such a composition are insufficient.

EP 1 853 249 A2 discloses a pharmaceutical composition comprising at least two active pharmaceutical ingredients, namely fenofibrate as a first ingredient and an HMG-CoA reductase inhibitor or a derivative thereof as a second ingredient. It provides a single solid dosage form for oral administration comprising a solid fenofibrate composition and a solid HMG-CoA reductase inhibitor composition, preferably a statin composition, the active substances being present in separate entities. The document discloses that the fibrate which is contained in the composition is present in the form of a solid solution. According to the examples disclosed, the fenofibrate granulate was prepared in a quite laborious way, namely according to the method of WO 2005/ 034 920 A1. This again means, that fenofibrate has to be dissolved in a solution of polyethylene glycol 6000 and poloxamer 188 (70:30 w/w ratio) at 75°C and the homogeneous solution has to be subsequently sprayed over lactose. Besides this drawback, the separation of the active substances in two entities, the dissolution profile of fenofibrate of the disclosed pharmaceutical composition is also unsatisfactory.

WO 2008/075320 A2 discloses process for preparing an oral pharmaceutical composition comprising fenofibrate alone or in combination with at least one other antilipidemic agent in a single dosage form that can be conveniently administered once or twice in a day. The document discloses that a process for preparing an oral pharmaceutical composition comprising fenofibrate, wherein the process comprises the steps of preparing a solution comprising fenofibrate, a surfactant and a hydrophilic polymer, and spraying the solution over one or more inert carriers, to prepare granules. This document also requires fenofibrate composition preparation by preparing solution of fenofibrate and using the solution to prepare granules.

Kumar et al., IJPSR, 2016; Vol. 7(1): 413-23 discloses combinational of rosuvastatin calcium and fenofibrate as bilayer tablet wherein rosuvastatin layer provides sustained release of rosuvastatin and fenofibrate layer provides immediate release of fenofibrate.

To summarize, the greatest part of the abovementioned prior art discloses single drug formulations of fenofibrate and rosuvastatin. Only a few prior art documents are directed to drug combinations of a fibrate and a statin in a single dosage form. Most notably, however, none of the prior art documents teaches how the dissolution of fenofibrate may be improved when both, fenofibrate and rosuvastatin, are combined in a single dosage form. Moreover, the known fixed-dose combinations are disadvantageous in terms of their manufacture. The preparation of a solid solution of the active substances introduces a high degree of complexity into the manufacturing process. In order to prepare such a solid solution, the manufacturer is obliged to use equipment for heating and add certain excipients (e.g. PEG, poloxamers, waxes).

Additionally, none of the prior art documents have shown to be effective in improving treatment compliance by providing a fixed dose combination of fenofibrate and rosuvastatin which is bioequivalent *in vivo* versus to a concomitant intake of monotherapies SECALIP SUPRA^{®} and CRESTOR^{®}.

In view of the prior art, there is a strong need for the development of a new combination product of fenofibrate and rosuvastatin which overcomes the disadvantages known from the conventional compositions in the prior art.

### OBJECT OF THE INVENTION

It is therefore the object of the current invention to provide a combination product of fenofibrate and rosuvastatin which can easily be manufactured and which has an improved dissolution profile, at least for fenofibrate.

### SUMMARY OF THE INVENTION

The inventors of the present invention have surprisingly found that a pharmaceutical composition comprising a fixed dose combination of rosuvastatin and fenofibrate or their pharmaceutically acceptable salts, wherein the composition has a multilayer structure, and the rosuvastatin and fenofibrate are found in separate layers, and wherein fenofibrate and rosuvastatin are immediately released from the fenofibrate layer and the rosuvastatin layer respectively, and fenofibrate layer comprises fenofibrate which is in the form of micronized fenofibrate, exhibit an excellent dissolution profile of fenofibrate. Preferably, the dissolution profile of fenofibrate is characterized by the fact that more than 74% of the fenofibrate content is dissolved within 30 minutes when measured in a type II dissolution apparatus USP, using 1000 ml 0.05M sodium lauryl sulphate in water as dissolution medium at 37°C and stirring at 50 revolutions per minute.

Advantageously, compositions of the present invention can be obtained by conventional wet granulation processes, *i.e.* in contrast to the fixed dose combinations of the prior art they do not require the preparation of a solid solution of fenofibrate. On the other hand, the compositions of the present invention exhibit a faster dissolution of fenofibrate than the fixed dose combinations of the prior art, *i.e.* they exhibit a dissolution profile such that more than 74% by weight of the fenofibrate is released within 30 minutes when measured in a type II dissolution apparatus USP, using 1000 ml 0.05M sodium lauryl sulphate in water as dissolution medium at 37°C and stirring at 50 revolutions per minute.

The pharmaceutical composition of the invention advantageously allows to replicate the *in vivo* bioavailability of the concomitant intake of commercially existing SECALIP SUPRA^{®} and CRESTOR^{®} at equivalent doses. In this sense, the inventors have found that the total drug exposure exhibited after the once-daily administration of the fixed dose combination of the present invention is bioequivalent to the total drug exposure obtained after the concomitant once-daily administration of a 160 mg tablet of SECALIP SUPRA^{®} and a 20 mg tablet of CRESTOR^{®}. This means that the fixed dose combination of the present invention shows comparable pharmacokinetic parameters on bioavailability (e.g. Cₘₐₓ, Tₘₐₓ, AUC₀₋ₜ, AUC_{0-∞}) of fenofibric acid, the active metabolite of fenofibrate, and rosuvastatin.

Surprisingly, due to the faster *in vitro* dissolution profile of fenofibrate of the multilayer pharmaceutical composition of the invention as compared to monolayer pharmaceutical compositions of the prior art, the multilayer pharmaceutical composition of the invention is able to replicate the *in vivo* bioavailability of SECALIP SUPRA^{®}.

Furthermore, the inventors have found that the multilayer pharmaceutical composition comprising a fixed dose combination of rosuvastatin and fenofibrate, or their pharmaceutically acceptable salts, shows a good stability of both active ingredients at 40 °C temperature and 75% relative humidity for time periods of 1 month, 2 months and 3 months.

Therefore, the multilayer pharmaceutical composition of the invention comprising a fixed dose combination of rosuvastatin and fenofibrate, or their pharmaceutically acceptable salts, represents a relevant improvement over the corresponding monotherapies, as it provides the two active ingredients within one single tablet for once-daily treatment, thus reducing the patient pill burden. Thus, the fixed dose-combination is convenient to mixed dyslipidemia patients which currently need to take the two monotherapies separately, and thus covers an unmet medical need and provides an improvement to ensure treatment compliance and adherence.

### BRIEF DESCRIPTION OF DRAWINGS

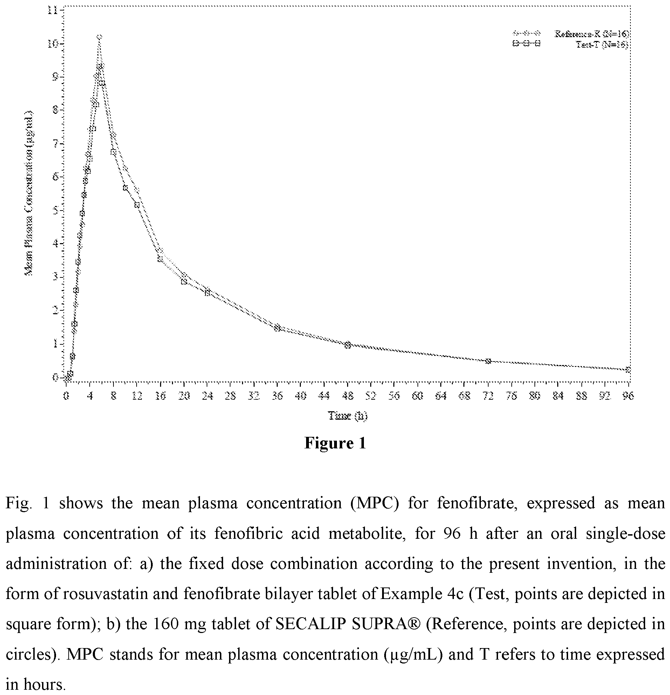

Fig. 1 shows the mean plasma concentration (MPC) for fenofibrate, expressed as mean plasma concentration of its fenofibric acid metabolite, for 96 h after an oral single-dose administration of: a) the fixed dose combination according to the present invention, in the form of rosuvastatin and fenofibrate bilayer tablet of Example 4c (Test, points are depicted in square form); b) the 160 mg tablet of SECALIP SUPRA^{®} (Reference, points are depicted in circles). MPC stands for mean plasma concentration (µg/mL) and T refers to time expressed in hours.

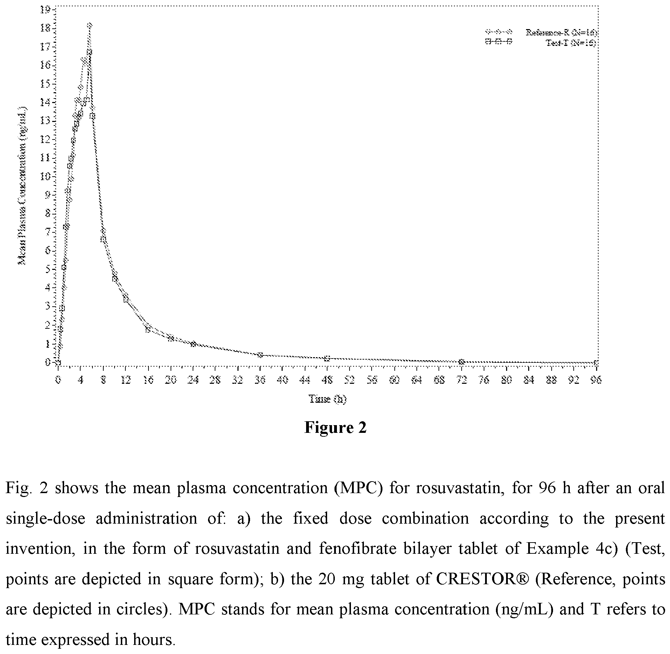

Fig. 2 shows the mean plasma concentration (MPC) for rosuvastatin, for 96 h after an oral single-dose administration of: a) the fixed dose combination according to the present invention, in the form of rosuvastatin and fenofibrate bilayer tablet of Example 4c) (Test, points are depicted in square form); b) the 20 mg tablet of CRESTOR^{®} (Reference, points are depicted in circles). MPC stands for mean plasma concentration (ng/mL) and T refers to time expressed in hours.

### DETAILED DESCRIPTION OF THE INVENTION

Unless otherwise indicated, terms in this specification are intended to have their ordinary meaning in the relevant art.

The present invention relates to compositions for the treatment of cardiovascular disease, including but not limited to, coronary heart disease, heart failure and cardiomyopathy, congenital heart disease, atherosclerosis, ischemia/reperfusion, hypertension, restenosis, acute rheumatic fever, rheumatic heart disease and arterial inflammation.

The present invention relates to pharmaceutical formulations with a synergistic effect of the combined use of a fenofibrate with a statin. Said formulations are presented in multiple dosage forms produced by formulating the two active agents separately. The excipients used in the formulations are selected such that the interaction between them and the active agents is minimized, and the formulations are prepared accordingly. Multiple dosage forms prepared this way can be produced such that the two active agents can have different and/or the same release characteristics.

As mentioned above, both fenofibrate and statin group active agents have solubility/dissolution problems. It is known that it is very difficult to develop a pharmaceutical form having a high dissolving speed of a hardly dissolving drug. Yet, the inventors have succeeded to produce multilayer composition comprising rosuvastatin and fenofibrate or pharmaceutically acceptable salts thereof wherein the dissolution of fenofibrate after 60 minutes is at least 20% higher compared to a composition comprising a monolayer of rosuvastatin or a pharmaceutically acceptable salt thereof and fenofibrate or a pharmaceutically acceptable salt thereof.

The term "rosuvastatin" as used herein defines the molecule represented by the structural formula (I) but also the pharmaceutically acceptable salts thereof. The multilayer composition of the invention comprises an amount of rosuvastatin calcium or, alternatively, an amount of another pharmaceutically acceptable salt equivalent to the amount of rosuvastatin calcium. Examples of such salts include barium salts, strontium salts, zinc salts, cesium salts, cadmium salts, magnesium salts, sodium salts and ammonium salts.

The term "fenofibrate" as used herein is defined as the molecule represented by the structural formula (II) but also as pharmaceutically acceptable salts thereof.

The present invention provides a multilayer, pharmaceutical composition comprising a fixed dose combination of rosuvastatin or a pharmaceutically acceptable salt thereof and fenofibrate or a pharmaceutically acceptable salt thereof and a process for preparation of the said multilayer composition.

In a preferred embodiment, the present invention provides a multilayer, pharmaceutical composition comprising a fixed dose combination of rosuvastatin or a pharmaceutically acceptable salt thereof and fenofibrate or a pharmaceutically acceptable salt thereof, wherein the multilayer composition is an immediate release bilayer tablet.

In another embodiment, the present invention provides a multilayer, pharmaceutical composition comprising a fixed dose combination of rosuvastatin or a pharmaceutically acceptable salt thereof and fenofibrate or a pharmaceutically acceptable salt thereof, wherein fenofibrate and rosuvastatin are immediately released from the fenofibrate layer and the rosuvastatin layer respectively, and dissolution of fenofibrate after 60 minutes from the multilayer composition comprising a fixed dose combination is at least 20% higher compared to a composition comprising a monolayer of rosuvastatin or a pharmaceutically acceptable salt thereof and fenofibrate or a pharmaceutically acceptable salt thereof.

In another embodiment, the present invention provides a multilayer, pharmaceutical composition comprising a fixed dose combination of rosuvastatin or a pharmaceutically acceptable salt thereof and fenofibrate or a pharmaceutically acceptable salt thereof, wherein fenofibrate and rosuvastatin are immediately released from the fenofibrate layer and the rosuvastatin layer respectively, and dissolution of fenofibrate after 60 minutes from the multilayer composition comprising a fixed dose combination is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In another embodiment, the present invention provides a multilayer, pharmaceutical composition comprising a fixed dose combination of rosuvastatin or a pharmaceutically acceptable salt thereof and fenofibrate or a pharmaceutically acceptable salt thereof, wherein fenofibrate and rosuvastatin are immediately released from the fenofibrate layer and the rosuvastatin layer respectively, and fenofibrate layer comprises micronized fenofibrate, and dissolution of fenofibrate after 60 minutes from the multilayer composition comprising a fixed dose combination is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In another embodiment, the present invention provides a bilayer tablet comprising fixed dose combination of rosuvastatin or a pharmaceutically acceptable salt thereof and fenofibrate or a pharmaceutically acceptable salt thereof, wherein dissolution of fenofibrate after 60 minutes from the bilayer tablet comprising a fixed dose combination is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In another embodiment, the present invention provides a multilayer, pharmaceutical composition comprising a fixed dose combination of rosuvastatin or a pharmaceutically acceptable salt thereof and fenofibrate or a pharmaceutically acceptable salt thereof, wherein rosuvastatin or the pharmaceutically acceptable salt thereof is present in an amount from 5 mg to 50 mg, and fenofibrate or the pharmaceutically acceptable salt is present in amount from 100 mg to 200 mg.

In a preferred embodiment, the present invention provides a bilayer tablet comprising a fixed dose combination of rosuvastatin or a pharmaceutically acceptable salt thereof and fenofibrate or a pharmaceutically acceptable salt thereof, wherein rosuvastatin or the pharmaceutically acceptable salt thereof is present in an amount from 5 mg to 20 mg, and fenofibrate or the pharmaceutically acceptable salt is present in amount from 140 mg to 180 mg.

In another embodiment, the present invention provides a multilayer, pharmaceutical composition comprising a fixed dose combination of rosuvastatin or a pharmaceutically acceptable salt thereof and fenofibrate or a pharmaceutically acceptable salt thereof, wherein (1) rosuvastatin or the pharmaceutically acceptable salt thereof is present in an amount from 5 mg to 20 mg, and fenofibrate or the pharmaceutically acceptable salt is present in amount from 140 mg to 180 mg and (2) rosuvastatin and fenofibrate are not mixed together, (3) fenofibrate and rosuvastatin are immediately released from the fenofibrate layer and the rosuvastatin layer, respectively and (4) dissolution of fenofibrate after 60 minutes from the multilayer composition is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In a preferred embodiment, the present invention provides a multilayer, pharmaceutical composition comprising a fixed dose combination of rosuvastatin or a pharmaceutically acceptable salt thereof and fenofibrate or a pharmaceutically acceptable salt thereof, wherein the multilayer composition comprises 5 to 20 mg rosuvastatin or a pharmaceutically acceptable salt and 160 mg fenofibrate, wherein (1) rosuvastatin and fenofibrate, are not mixed together, (2) fenofibrate and rosuvastatin are immediately released from fenofibrate layer and the rosuvastatin layer, respectively and (3) dissolution of fenofibrate after 15 minutes is more than 67 % w/w of the total fenofibrate content and (4) dissolution of fenofibrate after 60 minutes from the multilayer composition is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In another embodiment, the present invention provides a bilayer tablet comprising a fixed dose combination of rosuvastatin or a pharmaceutically acceptable salt thereof and fenofibrate or a pharmaceutically acceptable salt thereof, wherein rosuvastatin or the pharmaceutically acceptable salt thereof is present in an amount from 5 mg to 20 mg, and fenofibrate or the pharmaceutically acceptable salt is present in amount from 140 mg to 180 mg and wherein, dissolution of fenofibrate after 60 minutes from the bilayer tablet is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In a preferred embodiment, the present invention provides a bilayer tablet comprising a fixed dose combination of rosuvastatin or a pharmaceutically acceptable salt thereof and fenofibrate or a pharmaceutically acceptable salt thereof, wherein the bilayer tablet comprises 5 to 20 mg rosuvastatin or a pharmaceutically acceptable salt and 160 mg fenofibrate and/or wherein dissolution of fenofibrate after 60 minutes from the bilayer tablet is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In another embodiment, the present invention provides a bilayer tablet comprising a fixed dose combination of rosuvastatin or a pharmaceutically acceptable salt thereof and fenofibrate or a pharmaceutically acceptable salt thereof, wherein (1) rosuvastatin or the pharmaceutically acceptable salt thereof is present in an amount from 5 mg to 20 mg, and fenofibrate or the pharmaceutically acceptable salt is present in amount from 140 mg to 180 mg and (2) both rosuvastatin and fenofibrate are not mixed together, (3) fenofibrate and rosuvastatin are immediately released from the fenofibrate layer and the rosuvastatin layer, respectively and/or (4) dissolution of fenofibrate after 60 minutes from the bilayer tablet is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In a preferred embodiment, the present invention provides a bilayer tablet comprising a fixed dose combination of rosuvastatin or a pharmaceutically acceptable salt thereof and fenofibrate or a pharmaceutically acceptable salt thereof, wherein the bilayer tablet comprises 5 to 20 mg rosuvastatin or the pharmaceutically acceptable salt and 160 mg fenofibrate wherein; (1) both rosuvastatin and fenofibrate are not mixed together (i.e. not a monolayer), (2) fenofibrate and rosuvastatin are immediately released from the fenofibrate layer and the rosuvastatin layer, respectively and (3) dissolution of fenofibrate after 15 minutes is more than 67 % w/w of fenofibrate content and/or (4) dissolution of fenofibrate after 60 minutes from the bilayer tablet is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate and the rosuvastatin are present in separate layers and wherein the fenofibrate is micronized fenofibrate. In a preferred embodiment, the micronized fenofibrate has a volume median diameter D(v, 50) of more than 0.02 µm and less than or equal to 20 µm, preferably of more than 0.04 µm and less than or equal to 10 µm, more preferably of more than 0.05 µm and less than or equal to 7 µm, even more preferably of more than 0.05 µm and less than or equal to 5 µm, most preferably of more than 0.05 µm and less than or equal to 4 µm.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate and the rosuvastatin are present in separate layers and wherein the fenofibrate is micronized fenofibrate and wherein fenofibrate and rosuvastatin are immediately released from the fenofibrate layer and the rosuvastatin layer, respectively. In a preferred embodiment, the micronized fenofibrate has a volume median diameter D(v, 50) of more than 0.02 µm and less than or equal to 20 µm, preferably of more than 0.04 µm and less than or equal to 10 µm, more preferably of more than 0.05 µm and less than or equal to 7 µm, even more preferably of more than 0.05 µm and less than or equal to 5 µm, most preferably of more than 0.05 µm and less than or equal to 4 µm.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate and the rosuvastatin are present in separate layers and wherein the fenofibrate is micronized fenofibrate and wherein fenofibrate and rosuvastatin are immediately released from the fenofibrate layer and the rosuvastatin layer, respectively, and dissolution of fenofibrate after 60 minutes from the multilayer composition comprising a fixed dose combination is at least 20% higher compared to a composition comprising a monolayer of rosuvastatin or a pharmaceutically acceptable salt thereof and fenofibrate or a pharmaceutically acceptable salt thereof; when measured in a type II dissolution apparatus USP, using 1000 ml 0.05M sodium lauryl sulphate in water as dissolution medium at 37°C and stirring at 50 revolutions per minute. In a preferred embodiment, the micronized fenofibrate has a volume median diameter D(v, 50) of more than 0.02 µm and less than or equal to 20 µm, preferably of more than 0.04 µm and less than or equal to 10 µm, more preferably of more than 0.05 µm and less than or equal to 7 µm, even more preferably of more than 0.05 µm and less than or equal to 5 µm, most preferably of more than 0.05 µm and less than or equal to 4 µm.

In another embodiment, the micronized fenofibrate has a particle size distribution (PSD) such that D(v,10) is more than 0.02 µm and less than or equal to 7 µm, preferably more than 0.04 µm and less than or equal to 5 µm, more preferably more than 0.04 µm and less than or equal to 4 µm, even more preferably more than 0.05 µm and less than or equal to 3 µm, even more preferably more than 0.05 µm and less than or equal to 2 µm; and/or D(v,50) is more than 0.04 µm and less than or equal to 20 µm, preferably more than 0.08 µm and less than or equal to 10 µm, more preferably more than 0.1 µm and less than or equal to 7 µm, even more preferably more than 0.1 µm and less than or equal to 5 µm, even more preferably more than 0.1 µm and less than or equal to 4 µm; and/or D(v,90) is more than 0.05 µm and less than or equal to 47 µm, preferably more than 0.1 µm and less than or equal to 25 µm, more preferably more than 0.2 µm and less than or equal to 18 µm, even more preferably more than 0.4 µm and less than or equal to 13 µm, even more preferably more than 0.5 µm and less than or equal to 10 µm.

The width of the particle size distribution of the micronized fenofibrate, expressed as a span may be from 1.2 to 3, preferably from 1.3 to 2.5, more preferably from 1.6 to 2.3. According the Chew et al J Pharm Pharmaceut Sci 2002, 5, 162-168, the "span" corresponds to the following relation: [D(v,0.9) - D(v,0.1)]/D(v,0.5).

The PSD parameters (D(v,0.9), D(v,0.5) and D(v,0.1)) of the micronized fenofibrate are determined by measuring the characteristic equivalent sphere diameter, known as volume diameter, by laser diffraction.

In another embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate and the rosuvastatin are present in separate layers, and wherein the fenofibrate is not in the form of a solid solution or a solid dispersion. In this context, the fact that the fenofibrate is not in the form of a solid solution or a solid dispersion means that the fenofibrate layer comprises less than 20% w/w or is free of an excipient or carrier selected from the group consisting of polyethylene glycols, polypropylene glycols, polyoxyethylenes, polyoxypropylenes, poloxamers, straight chain saturated hydrocarbons, sorbitan esters, paraffins, fats, oils and mixtures thereof and having a melting point lower than 100°C. Preferably, the fenofibrate layer comprises less than 20% w/w or is free of an excipients or carrier with a melting point lower than 100°C irrespective of the class of compounds that the excipients or carrier is selected from. In a preferred embodiment, compositions of the invention comprise a fenofibrate layer that comprises less than 20% w/w or are free of an excipient or carrier with a melting point lower than 75°C, preferably lower than 80°C, more preferably lower than 90°C, even more preferably lower than 100°C.

In another embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate and the rosuvastatin are present in separate layers, and wherein fenofibrate and rosuvastatin are immediately released from the fenofibrate layer and the rosuvastatin layer, respectively and wherein the composition exhibits a dissolution profile according to which more than 74% w/w of fenofibrate is dissolved within 30 minutes, preferably more than 77% w/w, more preferably more than 80% w/w, even more preferably more than 83% w/w ; when measured in a type II dissolution apparatus USP, using 1000 ml 0.05M sodium lauryl sulphate in water as dissolution medium at 37°C and stirring at 50 revolutions per minute.

In another embodiment, the compositions of the invention exhibit a dissolution profile according to which: up to 48% w/w of fenofibrate is dissolved in 5 minutes, from 49 to 79% w/w of fenofibrate is dissolved in 10 minutes, from 65 to 95% w/w of fenofibrate is dissolved in 15 minutes, from 68 to 98% w/w of fenofibrate is dissolved in 20 minutes, from 74 to 100% w/w of fenofibrate is dissolved in 30 minutes, from 79 to 100% w/w of fenofibrate is dissolved in 45 minutes, and from 80 to 100% w/w of fenofibrate is dissolved in 60 minutes; when measured in a type II dissolution apparatus USP, using 1000 ml 0.05M sodium lauryl sulphate in water as dissolution medium at 37°C and stirring at 50 revolutions per minute. In another preferred embodiment, the compositions of the invention exhibit a dissolution profile according to which: up to 43% w/w of fenofibrate is dissolved in 5 minutes, from 54 to 74% w/w of fenofibrate is dissolved in 10 minutes, from 70 to 90% w/w of fenofibrate is dissolved in 15 minutes, from 73 to 93% w/w of fenofibrate is dissolved in 20 minutes, from 78 to 98% w/w of fenofibrate is dissolved in 30 minutes, from 84 to 100% w/w of fenofibrate is dissolved in 45 minutes, and from 85 to 100% w/w of fenofibrate is dissolved in 60 minutes; when measured in a type II dissolution apparatus USP, using 1000 ml 0.05M sodium lauryl sulphate in water as dissolution medium at 37°C and stirring at 50 revolutions per minute. In another more preferred embodiment, the compositions of the invention exhibit a dissolution profile according to which: up to 38% w/w of fenofibrate is dissolved in 5 minutes, from 59 to 69% w/w of fenofibrate is dissolved in 10 minutes, from 75 to 85% w/w of fenofibrate is dissolved in 15 minutes, from 78 to 88% w/w of fenofibrate is dissolved in 20 minutes, from 83 to 93% w/w of fenofibrate is dissolved in 30 minutes, from 89 to 99% w/w of fenofibrate is dissolved in 45 minutes, and from 90 to 100% w/w of fenofibrate is dissolved in 60 minutes; when measured in a type II dissolution apparatus USP, using 1000 ml 0.05M sodium lauryl sulphate in water as dissolution medium at 37°C and stirring at 50 revolutions per minute.

In another embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate and the rosuvastatin are present in separate layers, and wherein fenofibrate and rosuvastatin are immediately released from the fenofibrate layer and the rosuvastatin layer, respectively, and wherein the fenofibrate is micronized fenofibrate, and wherein the fenofibrate is not in the form of a solid dispersion.

In another embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate and the rosuvastatin are present in separate layers, and wherein fenofibrate and rosuvastatin are immediately released from the fenofibrate layer and the rosuvastatin layer, respectively, and wherein the fenofibrate is micronized fenofibrate, wherein the fenofibrate is not in the form of a solid dispersion, and wherein the composition exhibits a dissolution profile according to which more than 74% w/w of fenofibrate is dissolved within 30 minutes; when measured in a type II dissolution apparatus USP, using 1000 ml 0.05M sodium lauryl sulphate in water as dissolution medium at 37°C and stirring at 50 revolutions per minute.

In another embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate and the rosuvastatin are present in separate layers, and wherein fenofibrate and rosuvastatin are immediately released from the fenofibrate layer and the rosuvastatin layer, respectively, and wherein the fenofibrate is micronized fenofibrate, wherein the fenofibrate is not in the form of a solid dispersion, and wherein the composition exhibits a dissolution profile according to which: up to 48% w/w of fenofibrate is dissolved in 5 minutes, from 49 to 79% w/w of fenofibrate is dissolved in 10 minutes, from 65 to 95% w/w of fenofibrate is dissolved in 15 minutes, from 68 to 98% w/w of fenofibrate is dissolved in 20 minutes, from 73 to 100% w/w of fenofibrate is dissolved in 30 minutes, from 79 to 100% w/w of fenofibrate is dissolved in 45 minutes, and from 80 to 100% w/w of fenofibrate is dissolved in 60 minutes; when measured in a type II dissolution apparatus USP, using 1000 ml 0.05M sodium lauryl sulphate in water as dissolution medium at 37°C and stirring at 50 revolutions per minute.

In an embodiment, after an oral single-dose administration of the fixed dose combination of the present invention under fed conditions, fenofibrate or a pharmaceutically acceptable salt thereof, equivalent to 160 mg of fenofibrate, exhibits a maximum plasma concentration (Cₘₐₓ) of from 6.500 µg/mL to 12.500 µg/mL, expressed as plasma concentration of its fenofibric acid metabolite. In a preferred embodiment, after an oral single-dose administration of the fixed dose combination of the present invention under fed conditions, fenofibrate or a pharmaceutically acceptable salt thereof, equivalent to 160 mg of fenofibrate, exhibits a maximum plasma concentration (Cₘₐₓ) of from 8.000 µg/mL to 11.000 µg/mL, expressed as plasma concentration of its fenofibric acid metabolite. In a more preferred embodiment, after an oral single-dose administration of the fixed dose combination of the present invention under fed conditions, fenofibrate or a pharmaceutically acceptable salt thereof, equivalent to 160 mg of fenofibrate, exhibits a maximum plasma concentration (Cₘₐₓ) of from 9.000 µg/mL to 10.000 µg/mL, expressed as plasma concentration of its fenofibric acid metabolite. The term Cₘₐₓ refers to the maximum concentration of fenofibric acid metabolite in the blood following an oral single-dose administration of the fixed dose combination of the present invention under fed conditions.

In an embodiment, after an oral single-dose administration of the fixed dose combination of the present invention under fed conditions, rosuvastatin or a pharmaceutically acceptable salt thereof, equivalent to 20 mg of rosuvastatin, exhibits a maximum plasma concentration (Cₘₐₓ) of from 9.000 ng/mL to 29.000 ng/mL. In a preferred embodiment, after an oral single-dose administration of the fixed dose combination of the present invention under fed conditions, rosuvastatin or a pharmaceutically acceptable salt thereof, equivalent to 20 mg of rosuvastatin, exhibits a maximum plasma concentration (Cₘₐₓ) of from 13.000 ng/mL to 23.000 ng/mL. In a more preferred embodiment, after an oral single-dose administration of the fixed dose combination of the present invention under fed conditions, rosuvastatin or a pharmaceutically acceptable salt thereof, equivalent to 20 mg of rosuvastatin, exhibits a maximum plasma concentration (Cₘₐₓ) of from 17.000 ng/mL to 19.000 ng/mL. The term "Cₘₐₓ" refers to the maximum concentration of rosuvastatin in the blood following an oral single-dose administration of the fixed dose combination of the present invention under fed conditions.

In an embodiment, after an oral single-dose administration of the fixed dose combination of the present invention under fed conditions, fenofibrate or a pharmaceutically acceptable salt thereof, equivalent to 160 mg of fenofibrate, exhibits a time to maximum plasma concentration (Tₘₐₓ) of from 1.5 h to 7 h, expressed as plasma concentration of its fenofibric acid metabolite. In a preferred embodiment, after an oral single-dose administration of the fixed dose combination of the present invention under fed conditions, fenofibrate or a pharmaceutically acceptable salt thereof, equivalent to 160 mg of fenofibrate, exhibits a time to maximum plasma concentration (Tₘₐₓ) of from 5 h to 6 h, expressed as plasma concentration of its fenofibric acid metabolite. In a more preferred embodiment, after an oral single-dose administration of the fixed dose combination of the present invention under fed conditions, fenofibrate or a pharmaceutically acceptable salt thereof, equivalent to 160 mg of fenofibrate, exhibits a time to maximum plasma concentration (Tₘₐₓ) of from 5.25 h to 5.75 h, expressed as plasma concentration of its fenofibric acid metabolite. The term "Tₘₐₓ" refers to the time in hours when Cₘₐₓ is achieved following an oral single-dose administration of the fixed dose combination of the present invention under fed conditions.

In an embodiment, after an oral single-dose administration of the fixed dose combination of the present invention under fed conditions, rosuvastatin or a pharmaceutically acceptable salt thereof, equivalent to 20 mg of rosuvastatin, exhibits a time to maximum plasma concentration (Tₘₐₓ) of from 1 h to 7 h. In a preferred embodiment, after an oral single-dose administration of the fixed dose combination of the present invention under fed conditions, rosuvastatin or a pharmaceutically acceptable salt thereof, equivalent to 20 mg of rosuvastatin, exhibits a time to maximum plasma concentration (Tₘₐₓ) of from 4 h to 7 h. In a more preferred embodiment, after an oral single-dose administration of the fixed dose combination of the present invention under fed conditions, rosuvastatin or a pharmaceutically acceptable salt thereof, equivalent to 20 mg of rosuvastatin, exhibits a time to maximum plasma concentration (Tₘₐₓ) of from 5.25 h to 5.75 h. The term "Tₘₐₓ" refers to the time in hours when Cₘₐₓ is achieved following an oral single-dose administration of the fixed dose combination of the present invention under fed conditions.

In an embodiment, after an oral single-dose administration of the fixed dose combination of the present invention under fed conditions, fenofibrate or a pharmaceutically acceptable salt thereof, equivalent to 160 mg of fenofibrate, exhibits an area under the time/plasma concentration curve from time 0 to 96 hours (AUC (0-t)) of from 80.500 µg·h/mL to 310.500 µg·h/mL, expressed as plasma concentration of its fenofibric acid metabolite. In a preferred embodiment, after an oral single-dose administration of the fixed dose combination of the present invention under fed conditions, fenofibrate or a pharmaceutically acceptable salt thereof, equivalent to 160 mg of fenofibrate, exhibits an area under the time/plasma concentration curve from time 0 to 96 hours (AUC (0-t)) of from 122.500 µg·h/mL to 222.500 µg·h/mL, expressed as plasma concentration of its fenofibric acid metabolite. In a more preferred embodiment, after an oral single-dose administration of the fixed dose combination of the present invention under fed conditions, fenofibrate or a pharmaceutically acceptable salt thereof, equivalent to 160 mg of fenofibrate, exhibits an area under the time/plasma concentration curve from time 0 to 96 hours (AUC (0-t)) of from 147.500 µg·h/mL to 197.500 µg·h/mL, expressed as plasma concentration of its fenofibric acid metabolite. The term "AUC" refers to the area under the time/plasma concentration curve after an oral single-dose administration of the composition of the present invention. AUC 0-infinity denotes the area under the plasma concentration versus time curve from time 0 to infinity and AUCo-t denotes the area under the plasma concentration versus time curve from time 0 to time t.

In an embodiment, after an oral single-dose administration of the fixed dose combination of the present invention under fed conditions, rosuvastatin or a pharmaceutically acceptable salt thereof, equivalent to 20 mg of rosuvastatin, exhibits an area under the time/plasma concentration curve from time 0 to 96 hours (AUC (0-t)) of from 76.000 ng·h/mL to 210.000 ng·h/mL. In a preferred embodiment, after an oral single-dose administration of the fixed dose combination of the present invention under fed conditions, rosuvastatin or a pharmaceutically acceptable salt thereof, equivalent to 20 mg of rosuvastatin, exhibits an area under the time/plasma concentration curve from time 0 to 96 hours (AUC (0-t)) of from 101.000 ng·h/mL to 171.000 ng·h/mL. In a more preferred embodiment, after an oral single-dose administration of the fixed dose combination of the present invention under fed conditions, rosuvastatin or a pharmaceutically acceptable salt thereof, equivalent to 20 mg of rosuvastatin, exhibits an area under the time/plasma concentration curve from time 0 to 96 hours (AUC (0-t)) of from 126.000 ng·h/mL to 146.000 ng·h/mL. The term "AUC" refers to the area under the time/plasma concentration curve after an oral single-dose administration of the composition of the present invention. AUC0-infinity denotes the area under the plasma concentration versus time curve from time 0 to infinity and AUCo-t denotes the area under the plasma concentration versus time curve from time 0 to time t.

In an embodiment, after an oral single-dose administration of the fixed dose combination of the present invention under fed conditions, fenofibrate or a pharmaceutically acceptable salt thereof, equivalent to 160 mg of fenofibrate, exhibits a half-life (T1/2) from 12 h to 40 h, expressed as plasma concentration of its fenofibric acid metabolite. In a preferred embodiment, after an oral single-dose administration of the fixed dose combination of the present invention under fed conditions, fenofibrate or a pharmaceutically acceptable salt thereof, equivalent to 160 mg of fenofibrate, exhibits a half-life (T1/2) from 19 h to 25 h, expressed as plasma concentration of its fenofibric acid metabolite. The term "half-life (T1/2)" refers to the time taken by fenofibrate to lose half of its pharmacological activity.

In an embodiment, after an oral single-dose administration of the fixed dose combination of the present invention under fed conditions, rosuvastatin or a pharmaceutically acceptable salt thereof, equivalent to 20 mg of rosuvastatin, exhibits a half-life (T1/2) from 6 h to 44 h. In a preferred embodiment, after an oral single-dose administration of the fixed dose combination of the present invention under fed conditions, rosuvastatin or a pharmaceutically acceptable salt thereof, equivalent to 20 mg of rosuvastatin, exhibits a half-life (T1/2) from 10 h to 16 h. The term "half-life (T1/2)" refers to the time taken by rosuvastatin to lose half of its pharmacological activity.

The values of pharmacokinetic parameters after a single-unique dose of the fixed dose combination of the present invention under fed conditions as mentioned above (i.e. Cₘₐₓ, Tₘₐₓ, AUC₀₋ₜ, AUC_{0-∞}) indicate that the once-daily administration of the fixed dose combination of the present invention is bioequivalent to the total drug exposure obtained after once-daily administration of the concomitant intake of a 160 mg tablet of SECALIP SUPRA^{®} and a 20 mg tablet of CRESTOR^{®}. The 90% confidence interval for the ratio of pharmacokinetic parameters of the fixed dose combination of the present invention and the comparative composition falling outside the scope of the present invention is contained within the acceptance interval of 80.00-125.00% according to the EMA Guideline on the investigation of bioequivalence (CPMP/EWP/QWP/1401/98 Rev. 1/ Corr, 2010).

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate or a pharmaceutically acceptable salt thereof and the rosuvastatin or a pharmaceutically acceptable salt thereof are present in separate layers, and wherein fenofibrate and rosuvastatin are immediately released from the fenofibrate layer and the rosuvastatin layer, respectively, and wherein the impurity-B of fenofibrate is below quantification limit under the storage conditions of 40 °C temperature and 75% relative humidity after a time period of 3 months.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate or a pharmaceutically acceptable salt thereof and the rosuvastatin or a pharmaceutically acceptable salt thereof are present in separate layers, and wherein fenofibrate and rosuvastatin are immediately released from the fenofibrate layer and the rosuvastatin layer, respectively, and wherein the fenofibrate assay is not less than 99.00% under the storage conditions of 40 °C temperature and 75% relative humidity after a time period of 3 months.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate or a pharmaceutically acceptable salt thereof and the rosuvastatin or a pharmaceutically acceptable salt thereof are present in separate layers, and wherein fenofibrate and rosuvastatin are immediately released from the fenofibrate layer and the rosuvastatin layer, respectively, and wherein the total amount of impurities related to fenofibrate is below 0.100 % under the storage conditions of 40 °C temperature and 75% relative humidity after a time period of 3 months.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate or a pharmaceutically acceptable salt thereof and the rosuvastatin or a pharmaceutically acceptable salt thereof are present in separate layers, and wherein fenofibrate and rosuvastatin are immediately released from the fenofibrate layer and the rosuvastatin layer, respectively, and wherein the anti-isomer impurity of rosuvastatin is below 0.250 % under the storage conditions of 40 °C temperature and 75% relative humidity after a time period of 3 months.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate or a pharmaceutically acceptable salt thereof and the rosuvastatin or a pharmaceutically acceptable salt thereof are present in separate layers, and wherein fenofibrate and rosuvastatin are immediately released from the fenofibrate layer and the rosuvastatin layer, respectively, and wherein the 5-oxo impurity of rosuvastatin is below 0.300 % under the storage conditions of 40 °C temperature and 75% relative humidity after a time period of 3 months.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate or a pharmaceutically acceptable salt thereof and the rosuvastatin or a pharmaceutically acceptable salt thereof are present in separate layers, and wherein fenofibrate and rosuvastatin are immediately released from the fenofibrate layer and the rosuvastatin layer, respectively, and wherein the lactone impurity of rosuvastatin is below 0.150 % under the storage conditions of 40 °C temperature and 75% relative humidity after a time period of 3 months.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate or a pharmaceutically acceptable salt thereof and the rosuvastatin or a pharmaceutically acceptable salt thereof are present in separate layers, and wherein fenofibrate and rosuvastatin are immediately released from the fenofibrate layer and the rosuvastatin layer, respectively, and wherein the rosuvastatin assay is not less than 99.00% under the storage conditions of 40 °C temperature and 75% relative humidity after a time period of 3 months.

In an embodiment, the present invention provides a multilayer pharmaceutical composition comprising a fixed dose combination of rosuvastatin or a pharmaceutically acceptable salt thereof and fenofibrate or a pharmaceutically acceptable salt thereof, wherein the fenofibrate or a pharmaceutically acceptable salt thereof and the rosuvastatin or a pharmaceutically acceptable salt thereof are present in separate layers, and wherein fenofibrate and rosuvastatin are immediately released from the fenofibrate layer and the rosuvastatin layer, respectively, and wherein the total amount of impurities related to rosuvastatin is below 0.700 % under the storage conditions of 40 °C temperature and 75% relative humidity after a time period of 3 months.

In more detailed aspects, the present invention provides a multilayer, pharmaceutical composition comprising a fixed dose combination of rosuvastatin or a pharmaceutically acceptable salt thereof and fenofibrate or a pharmaceutically acceptable salt thereof and suitable pharmaceutically acceptable excipients, wherein the pharmaceutically acceptable excipients are selected form one or more fillers or diluents, one or more binders, one or more disintegrants, one or more lubricants, one or more film coating agents, one or more pigments or plasticizers and the like.

For the purpose of this specification, the term "immediate release" means a composition or component or layer or unit comprising rosuvastatin and fenofibrate which releases rosuvastatin and fenofibrate from the composition or component or layer or unit immediately i.e. not according to a delayed or extended release mechanism. The multilayer, pharmaceutical composition according to present invention provides immediate release of fenofibrate and rosuvastatin from the fenofibrate layer and the rosuvastatin layer, respectively.

Pharmaceutical excipients for the present invention are selected from one or more fillers, one or more diluents, one or more binders, one or more disintegrants, one or more lubricants, one or more glidants, one or more surfactants/wetting agents, one or more film coating agents, one or more pigments or plasticizers, and the like.

According to present invention, fillers or diluents may include, but may not be limited to, ammonium alginate, calcium carbonate, calcium lactate, calcium phosphate dibasic anhydrous, calcium phosphate dibasic dihydrate, calcium phosphate tribasic, calcium silicate, calcium sulfate, microcrystalline cellulose, cellulose powdered, cellulose silicified microcrystalline, cellulose acetate, corn starch and pregelatinized starch, dextrates, dextrin, dextrose, erythritol, ethylcellulose, fructose, fumaric acid, glyceryl palmitostearate, isomalt, kaolin, lactitol, lactose anhydrous, lactose monohydrate, lactose monohydrate and corn starch, lactose monohydrate and povidone, lactose monohydrate and lactose spray-dried, lactose monohydrate and microcrystalline cellulose, magnesium carbonate, magnesium oxide, maltitol, maltodextrin, maltose, mannitol, polydextrose, polymethacrylates, simethicone, sodium alginate, sodium chloride, sorbitol, starch, starch pregelatinized, starch sterilizable maize, sucrose, sugar compressible, sugar confectioner's, sugar spheres, sulfobutylether b-cyclodextrin, sunflower oil, talc, tragacanth, trehalose, xylitol and mixtures thereof.

According to present invention, binders may include, but may not be limited to, acacia, agar, alginic acid, calcium carbonate, calcium phosphate tribasic, calcium lactate, carbomer, carboxymethylcellulose sodium, carrageenan, cellulose acetate phthalate, ceratonia, cellulose microcrystalline, chitosan, copovidone, cottonseed oil, dextrates, dextrin, dextrose, ethylcellulose, gelatin, liquid glucose, glyceryl behenate, guar gum, hydroxyethyl cellulose, hydroxyethylmethyl cellulose, hydroxypropyl cellulose, hydroxypropyl starch, lactose anhydrous, spray-dried lactose, low-substituted hydroxypropyl cellulose, hypromellose, inulin, monohydrate lactose, magnesium aluminum silicate, maltodextrin, maltose, methylcellulose, pectin, poloxamer, polycarbophil, polydextrose, polyethylene oxide, polymethacrylates, povidone, sodium alginate, starch pregelatinized, starch, sucrose, sunflower oil, hydrogenated vegetable oil, vitamin e, polyethylene glycol succinate, zein, tragacanth, polyethylene glycol, polyvinylpyrrolidone, stearic acid, tricaprylin and mixtures thereof.

According to present invention, disintegrants may include, but may not be limited to, alginic acid, calcium alginate, carboxymethylcellulose calcium, carboxymethylcellulose sodium, cellulose, cellulose microcrystalline, cellulose powdered, chitosan, colloidal silicon dioxide, corn starch and pregelatinized starch, croscarmellose sodium, crospovidone, docusate sodium, glycine, guar gum, hydroxypropyl cellulose low-substituted, hydroxypropyl starch, lactose, monohydrate and corn starch, magnesium aluminum silicate, methylcellulose, polacrilin potassium, povidone, sodium alginate, sodium starch glycolate, starch, starch pregelatinized and mixtures thereof.

According to present invention, lubricants may include, but may not be limited to, calcium stearate, canola oil, castor oil, hydrogenated, glyceryl behenate, glyceryl monostearate, glyceryl palmitostearate, lauric acid, leucine, magnesium stearate, light mineral oil, mineral oil, magnesium lauryl sulfate, myristic acid, octyldodecanol, palmitic acid, poloxamer, polyethylene glycol, polyvinyl alcohol, potassium benzoate, sodium benzoate, sodium hyaluronate, sodium lauryl sulfate, sodium stearyl fumarate, spray-dried lactose, starch sterilizable maize, stearic acid, talc, tricaprylin, vegetable oil hydrogenated, zinc stearate, sodium chloride and mixtures thereof.

According to present invention, glidants may include, but may not be limited to, tribasic calcium phosphate, powdered cellulose, colloidal silicon dioxide, hydrophobic colloidal silica, magnesium oxide, magnesium trisilicate, magnesium silicate, silicon dioxide, talc and mixtures thereof.

According to present invention, the surfactants/wetting agents may include, but may not be limited to, benzalkonium chloride, benzethonium chloride, cetylpyridinium chloride, docusate sodium, glycine, glycofurol, hypromellose, poloxamer, phospholipids, polyoxyethylene alkyl ethers, polyoxyethylene castor oil derivatives, polyoxyethylene sorbitan fatty acid esters, polyoxyethylene stearates, sodium lauryl sulfate, sorbitan esters (sorbitan fatty acid esters), tricaprylin and mixtures thereof.

The dose and dosing ratio of rosuvastatin calcium and fenofibrate can be adapted based various factors such as symptoms, age and body weight of the patients. According to present invention, the dosage of the rosuvastatin calcium is typically from 0.1 to 100 mg, in particular from 1 to 50 mg. More particular, unit dosage strengths of rosuvastatin calcium for inclusion into a multilayer, pharmaceutical composition of the present invention are 5, 10 or 20 mg.

According to present invention, the unit dosage strengths of the fenofibrate for use in the present invention may be from 10 mg to 500 mg, preferably from 50 mg to 350 mg. More particular, the unit dosage strength of fenofibrate for inclusion into a multilayer, pharmaceutical composition of the present invention of the present invention is 160 mg.

According to present invention, the multilayer, pharmaceutical composition comprising a fixed dose combination may be administered once or twice daily to the patient.

In a more preferred aspect of the invention; the present invention provides a process for the preparation of a multilayer, pharmaceutical composition comprising fixed dose combination of rosuvastatin calcium, fenofibrate and one or more pharmaceutical acceptable excipients, which are described / exemplified herein.

In a particular preferred embodiment, the dosage form of the present invention is a solid dosage form such as a tablet, a capsule, a powder and a sachet, but preferably a tablet.

The pharmaceutical composition described herein may be prepared by conventional technology well known to those skilled in the art.

A typical bilayer tablet (rosuvastatin calcium and fenofibrate) of this invention comprises one or more fillers or diluents (such as e.g. lactose, microcrystalline cellulose), one or more binders (such as e.g. povidone, microcrystalline cellulose, lactose etc.), one or more disintegrants (such as e.g. microcrystalline cellulose, croscarmellose sodium colloidal silicon dioxide, crospovidone) one or more lubricants (such as e.g. magnesium stearate), one or more glidants (such as e.g. tribasic calcium phosphate, colloidal silicon dioxide and magnesium oxide) and/or one or more surfactant (such as e.g. sodium lauryl sulfate).

According to the present invention a "multilayer pharmaceutical composition" is a multilayer tablet. The multilayer tablet may, for example be a bilayer tablet.

According to present invention "bilayer tablet" means a tablet comprising rosuvastatin or pharmaceutically acceptable salt thereof and fenofibrate, wherein rosuvastatin and fenofibrate are present in separate layers (i.e. rosuvastatin and fenofibrate are not mixed together during preparation of the composition).

According to present invention, "wet granulation" involves the addition of a granulating fluid, commonly water, functioning as a granulating liquid, to a stirred powder comprising the materials to be granulated.

According to present invention, "direct compression" involves a method of tablet production in which dry ingredients are thoroughly mixed and then compressed into tablets.

According to present invention, "dissolution" refers to the rate of drug release from a dosage form which may be measured by standardized methods.

According to present invention, the term "multilayer pharmaceutical composition" means a pharmaceutical composition having at least two layers. Examples of a multilayer pharmaceutical composition are, but are not limited to: multilayer tablets, such as bilayer tablets, tri-layer tablets, multi-coated tablets, compressed pellets, compressed coated pellets, multiple unit pellet system (MUPS), tablet-in-tablet, tablet(s)-in-capsule, compression coated tablets and the like.

Hereinafter the process of manufacturing the pharmaceutical composition of the present invention will be explained in detail:

### EXAMPLES

In order to further illustrate the present invention, the following examples are provided for the purpose of clarity of understanding. However, it is not intended in any way to limit the scope of present invention and it is readily apparent to those of ordinary skill in the art in light of the teachings of this invention that certain changes and modifications may be made thereto without departing from the scope of the invention.

### Preparation of fenofibrate (micronized):

For comparative example 1 and example 2 - 4 fenofibrate has been micronized to have a particle distribution characterized by the PSD parameters listed in table 1 below.

**Table 1: Particle size distribution parameters of micronized fenofibrate, wherein NMT means "not more than".**

| Parameter | Limits |
|---|---|
| D(v, 10) | NMT 2 µm |
| D(v, 50) | NMT 4 µm |
| D(v, 90) | NMT 10 µm |

### Comparative example 1: Rosuvastatin and fenofibrate monolayer tablets (not according to the invention)

**Table 2: Composition of a conventional rosuvastatin/fenofibrate monolayer tablet with a combined dose of 5+160 mg/tablet.**

| | Strength | 5+160 mg/tablet |
|---|---|---|
| **Sr. No.** | **Ingredients** | **mg/tab** |
| 1 | Fenofibrate (Micronized) | 160.00 |
| 2 | Lactose | 138.00 |
| 3 | Microcrystalline cellulose | 217.00 |
| 4 | Croscarmellose sodium | 23.00 |
| 5 | Crospovidone | 11.00 |
| 6 | Sodium lauryl sulphate | 6.00 |
| 7 | Ferric oxide red | 1.50 |
| 8 | Povidone k30 | 21 |
| 9 | Purified water^{∗∗} | q.s. |
| 10 | Isopropyl alcohol^{∗∗} | q.s. |
| 11 | Rosuvastatin calcium | 5.21 ^{∗} |
| 12 | Micro crystalline cellulose | 137.29 |
| 13 | Crospovidone | 7.00 |
| 14 | Light magnesium oxide | 8.00 |
| 15 | Colloidal anhydrous silica | 7.00 |
| 16 | Sodium stearyl fumarate | 8.00 |
| 17 | Opadry II for film coating | 22.5 |
| | **Total weight of tablet in mg** | **772.5** |

| | | |
|---|---|---|
| ^{∗} - 5.21 mg rosuvastatin calcium is equivalent to 5 mg rosuvastatin. ^{∗∗} - Not present in final composition. | | |

### Manufacturing process:

Fenofibrate granules were prepared by wet granulation process using the ingredients listed above under serial numbers Sr. No 1 to 9. A rosuvastatin blend was prepared by mixing rosuvastatin calcium with microcrystalline cellulose, crospovidone, colloidal anhydrous silica and light magnesium oxide. Subsequently, the fenofibrate granules and the rosuvastatin blend were mixed homogeneously. Sodium stearyl fumarate was added to the mixture of the fenofibrate granules and the rosuvastatin blend and the lubricated blend was compressed using suitable tools with a monolayer compression machine. The obtained monolayer tablet was film coated using Opadry.

### Example 2: Rosuvastatin and fenofibrate bilayer tablets (according to present invention)

**Table 3: Weight ratios of the ingredients in two layers of the inventive rosuvastatin/fenofibrate multilayer tablet. The inventive multilayer tablet is, for instance, available in a combined dose of 5+160 mg/tablet, 10+160 mg/tablet or 20+160 mg/tablet.**

| **Sr. No.** | **Ingredients** | |
|---|---|---|
| **Layer 1 Fenofibrate Layer** | | % **wt/wt** |
| 1 | Fenofibrate (Micronized) | 5-50% |
| 2 | Lactose monohydrate | 5-50% |
| 3 | Microcrystalline cellulose | 2-80% |
| 4 | Croscarmellose sodium | 2-15% |
| 5 | Crospovidone | 0.2-9% |
| 6 | Sodium lauryl sulphate | 0.2-3% |
| 7 | Ferric oxide red | 0.001-1% |
| 8 | Povidone k-30 | 0.5-5% |
| 9 | Purified water (80%)^{∗∗} | q.s. |
| 10 | Isopropyl alcohol (20%)^{∗∗} | q.s. |
| 11 | Crospovidone | 0.2-9% |
| 12 | Croscarmellose sodium | 2-15% |
| 13 | Colloidal anhydrous silica | 0.001-1% |
| 14 | Sodium stearyl fumarate | 0.1-5% |

| **Layer 2 Rosuvastatin Layer** | | |
|---|---|---|
| 1 | Rosuvastatin Calcium | 0.1-10% |
| 2 | Lactose anhydrous | 5-50% |
| 3 | Microcrystalline cellulose | 2-50% |
| 4 | Crospovidone | 0.2-5% |
| 5 | Light magnesium oxide | 0.001-5% |
| 6 | Magnesium stearate | 0.001-1% |

| | | |
|---|---|---|
| ^{∗∗} - Not present in final composition. | | |

### Manufacturing process:

The manufacturing Process is the same as described below in Example 3:

### Example 3: Rosuvastatin and fenofibrate bilayer tablets (according to present invention)

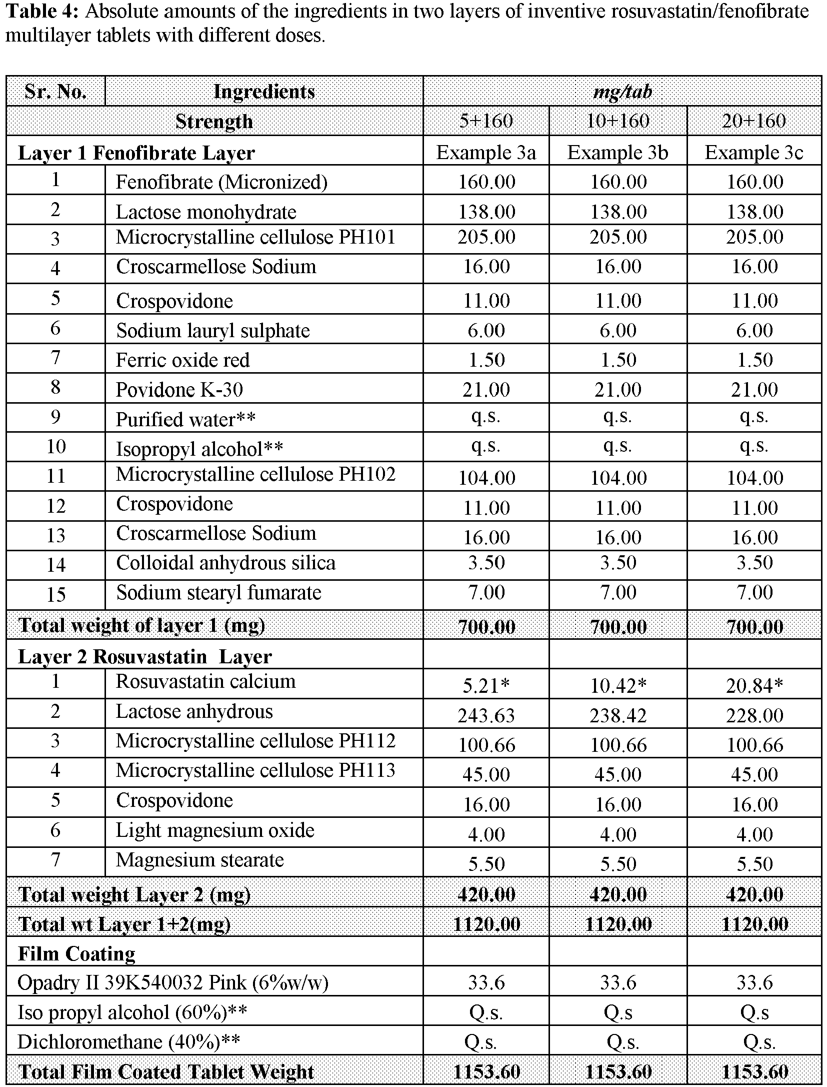

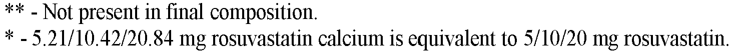

### Manufacturing process:

### Layer-1 (Fenofibrate Layer):

A fenofibrate blend was prepared by sifting the intragranular ingredients listed above under serial numbers Sr. No 1 to 7 through a suitable sieve and by loading it in a suitable granulator. A binder solution was prepared by dissolving povidone (Sr. No 8) in purified water adding isopropyl alcohol to obtain a suitable mixture. Fenofibrate granules were obtained by granulating the fenofibrate blend (Sr. No 1-7) with the granulating solvent in the suitable granulator. Subsequently, obtained granules and extragranular ingredients listed above under serial numbers Sr. No 11 to 14 were mixed homogenously. Finally, sodium stearyl fumarate (Sr. No 15) was added to the mixture to obtain a lubricated fenofibrate layer blend.

### Layer-2 (Rosuvastatin layer):

A rosuvastatin blend was prepared by mixing rosuvastatin calcium, lactose anhydrous, microcrystalline cellulose, crospovidone and light magnesium oxide in a blender. Sifted magnesium stearate was mixed with the previously prepared rosuvastatin blend to obtain a lubricated blend.

### Bilayer tablet:

The lubricated blends of fenofibrate and rosuvastatin were compressed using suitable tools with a bilayer compression machine. The obtained bilayer tablet was film coated using Opadry.

### Dissolution study:

The dissolution studies were carried out for:
- Innovator sample of fenofibrate (Supralip^{®}), a 160 mg film coated tablet
- Comparative example 1, monolayer tablet - not according to the invention
- Example 3a, Bilayer tablet - according to the invention

The dissolution studies were carried out using the following parameters:
Medium: 0.05M SLS in water, Apparatus: Paddle Type, Volume: 1000 ml, Speed: 50 RPM (Revolutions per minute)

The obtained dissolution profile results of fenofibrate is shown in table 5 below.

**Table 5: Dissolution study results of fenofibrate of Example 3a**

| **Parameters** | Supralip^{®} (Innovator) % **Drug release (Fenofibrate)** | Comparative example 1 **(Monolayer tablet)** | Example 3a **(Bilayer tablet)** | % **difference* in Dissolution** |
|---|---|---|---|---|
| Time Points | | | | |
| 5 | 20 | 30 | 33 | + 10.00 |
| 10 | 54 | 52 | 64 | + 23.08 |
| 15 | 81 | 62 | 80 | + 29.03 |
| 20 | 92 | 68 | 83 | + 22.06 |
| 30 | 94 | 71 | 88 | + 23.94 |
| 45 | 99 | 74 | 94 | + 27.03 |
| **60** | 102 | 75 | 95 | + **26.67** |

| | | | | |
|---|---|---|---|---|
| ^{∗} - The difference in dissolution is between Comparative example 1 and Example 3 a | | | | |

The above dissolution data provide evidence that the dissolution of fenofibrate after 60 minutes is at least 20% higher for the bilayer tablet with micronized fenofibrate than for the monolayer tablet of comparative example 1. Thus, the present invention allows providing a multilayer, pharmaceutical composition comprising a fixed dose combination of rosuvastatin calcium and fenofibrate which has a higher dissolution of fenofibrate after 60 minutes than conventional monolayer compositions.

### Example 4: Rosuvastatin and fenofibrate bilayer tablets (according to present invention)

| **Strength** | *5*/*160 mg* | *10*/*160 mg* | *20*/*160 mg* |
|---|---|---|---|
| **Layer 1 Fenofibrate Layer** | **Example 4a** | **Example 4b** | **Example 4c** |
| Fenofibrate Ph. Eur. | 160 | 160 | 160 |
| Lactose Monohydrate | 138 | 138 | 138 |
| Micro crystalline Cellulose(Avicel PH 101) | 205.3 | 205.3 | 205.3 |
| Croscarmellose Sodium | 21 | 21 | 21 |
| Crospovidone | 11 | 11 | 11 |
| Ferric Oxide Red | 1.5 | 1.5 | 1.5 |
| Sodium lauryl Sulphate | 5.7 | 5.7 | 5.7 |
| Povidone K30 | 40 | 40 | 40 |
| Purified water^{∗∗} | Q.S. | Q.S. | Q.S. |
| Isopropyl Alcohol^{∗∗} | Q.S. | Q.S. | Q.S. |
| Anhydrous Lactose | 92 | 92 | 92 |
| Crospovidone | 8 | 8 | 8 |
| Croscarmellose Sodium | 7 | 7 | 7 |
| Colloidal Anhydrous Silica | 3.5 | 3.5 | 3.5 |
| Sodium Stearyl Fumarate | 7 | 7 | 7 |
| Total weight of Layer 1 (mg) | 700 | 700 | 700 |
| **Layer 2 Rosuvastatin Layer** | | | |
| Rosuvastatin Calcium Ph. Eur. | 5.21 | 10.42 | 20.84 |
| Anhydrous Lactose | 210 | 204.79 | 194.37 |
| Micro Crystalline Cellulose (Avicel PH112) | 129.29 | 129.29 | 129.29 |
| Microcrystalline Cellulose (Avicel PH113) | 50 | 50 | 50 |
| Crospovidone | 16 | 16 | 16 |
| Light Magnesium Oxide | 4 | 4 | 4 |
| Magnesium Stearate | 5.5 | 5.5 | 5.5 |
| Total weight of Layer 2 (mg) | 420 | 420 | 420 |
| **Total weight of Layer 1+2 (mg)** | **1120** | **1120** | **1120** |
| **Film Coating** | | | |
| Opadry II 39K565030 Brown | 33.6 | --- | --- |
| Opadry II 39K565027 Brown | --- | 33.6 | --- |
| Opadry II 39K540032 Pink | --- | --- | 33.6 |
| Isopropyl Alcohol^{∗∗} | Q.S. | Q.S. | Q.S. |
| Dichloromethane^{∗∗} | Q.S. | Q.S. | Q.S. |
| **Total Film Coated Tablet Weight** | **1153.6** | **1153.6** | **1153.6** |

| | | | |
|---|---|---|---|
| ^{∗∗} - Not present in final composition. ^{∗} - 5.21/10.42/20.84 mg rosuvastatin calcium is equivalent to 5/10/20 mg rosuvastatin. | | | |

### Manufacturing process:

The manufacturing Process is the similar as described in Example 3:

### Dissolution study: The dissolution studies were carried out using the same parameters given above in example 3

**Table 6: Dissolution study results of fenofibrate of Example 4b and Example 4c**

| **Time Points** | Comparative example 1 (Monolayer tablet) | Example 4b (Bilayer tablet) | % difference* in dissolution | Example 4c (Bilayer tablet) | % difference* in dissolution |
|---|---|---|---|---|---|
| 5 | 30 | 25 | -16.67 | 33 | 10.00 |
| 10 | 52 | 53 | 1.92 | 68 | 30.77 |
| 15 | 62 | 72 | 16.13 | 83 | 33.87 |
| 20 | 68 | 81 | 19.12 | 89 | 30.88 |
| 30 | 71 | 88 | 23.94 | 95 | 33.80 |
| 45 | 74 | 93 | 25.68 | 99 | 33.78 |
| **60** | 75 | 94 | 25.33 | 100 | 33.33 |

| | | | | | |
|---|---|---|---|---|---|
| ^{∗} - The difference in dissolution is between Comparative example 1 and Example 4b / 4c | | | | | |

### Stability study:

The stability study were carried out for bilayer tablets obtained in Example 4a, Example 4b and Example 4c at 40 °C temperature and 75% relative humidity for time periods of 1 month, 2 months and 3 months. The obtained stability study results are shown in table 7a and table 7b below.

**Table 7a: stability study results for rosuvastatin**

| Examples | Timeline | Rosuvastatin Dissolution at 30 minutes | Rosuvastatin Assay | Rosuvastatin Related substances | | | | |
|---|---|---|---|---|---|---|---|---|
| | | | | Anti-isomer % (NMT 0.5%) | 5-oxo impurity % (NMT 0.5%) | Lactone impurity % (NMT 0.5%) | Single max unknown impurity %(NMT 0.2%) | Total impurities % (NMT 1.5%) |
| Example 4a | Initial | 103% | 100.30% | 0.001 | 0.116 | ND | 0.037 | 0.340 |
| | 1 month | 99% | 100.90% | ND | 0.166 | 0.017 | 0.033 | 0.404 |
| | 2 month | 100% | 100.50% | 0.101 | 0.125 | 0.043 | 0.046 | 0.455 |
| | 3 month | 102% | 100.90% | 0.141 | 0.132 | 0.046 | 0.041 | 0.419 |
| Example 4b | Initial | 103% | 100.10% | 0.140 | ND | ND | 0.020 | 0.354 |
| | 1 month | 101% | 97.90% | 0.115 | 0.134 | 0.047 | 0.030 | 0.424 |
| | 2 month | 99% | 100.10% | 0.139 | 0.166 | 0.040 | 0.039 | 0.480 |
| | 3 month | 101% | 99.50% | 0.219 | 0.175 | 0.025 | 0.028 | 0.519 |
| Example 4c | Initial | 97% | 99.10% | 0.227 | 0.040 | ND | 0.038 | 0.446 |
| | 1 month | 98.00% | 99.70% | 0.102 | 0.201 | 0.035 | 0.053 | 0.428 |
| | 2 month | 97.00% | 99.10% | 0.106 | 0.203 | 0.050 | 0.044 | 0.467 |
| | 3 month | 100% | 100.20% | 0.132 | 0.248 | 0.098 | 0.040 | 0.618 |

**Table 7b: stability study results for fenofibrate**

| Examples | | Fenofibrate Dissolution at 30 minutes | Fenofibrate Assay | Fenofibrate Related substances | | |
|---|---|---|---|---|---|---|
| | | | | Impurity B% (NMT 0.2%) | Single Max Unknown Impurity % (NMT 0.2%) | Total impurities % (NMT 1.0%) |
| Example 4a | Initial | 87% | 101.50% | ND | 0.011 | 0.014 |
| | 1 month | 91% | 100.50% | 0.001 | 0.011 | 0.016 |
| | 2 month | 92% | 99.80% | ND | 0.010 | 0.013 |
| | 3 month | 92% | 100.50% | ND | 0.011 | 0.015 |
| Example 4b | Initial | 88% | 101.40% | 0.002 | 0.011 | 0.020 |
| | 1 month | 94% | 99.10% | ND | 0.010 | 0.010 |
| | 2 month | 89% | 101.70% | ND | 0.012 | 0.020 |
| | 3 month | 91% | 99.50% | ND | 0.011 | 0.020 |
| Example 4c | Initial | 95% | 101.40% | ND | 0.011 | 0.014 |
| | 1 month | 91% | 101.70% | 0.002 | 0.011 | 0.017 |
| | 2 month | 90% | 101.60% | ND | 0.011 | 0.014 |
| | 3 month | 91% | 100.30% | ND | 0.011 | 0.015 |

From the stability data provided in the Table 7a and Table 7b, it can be concluded that the multilayer, pharmaceutical composition comprising a fixed dose combination of the present invention is found stable.

### Example 5: Bioavailability Test (according to present invention)

The bioavailability of the fixed dose combination according to the present invention, in the form of rosuvastatin and fenofibrate bilayer tablets was studied.

### Design of the study

The bioequivalence study was carried as per existing European Medicines Agency bioequivalence guideline.

### Samples

The samples used in the bioavailability test are the following:
a) Test sample: The fixed dose combination according to the present invention, in the form of rosuvastatin and fenofibrate bilayer tablet of 4c
b) Reference sample: SECALIP SUPRA^{®} 160 mg tablet and CRESTOR^{®} 20 mg tablet.

### Determinations

The method involved the measurement of the plasma concentration of fenofibric acid, the active metabolite of fenofibrate, and rosuvastatin, for 96 hours at predetermined time points.

Mean plasma concentration of fenofibric acid metabolite and rosuvastatin were determined. Values of fenofibric acid concentration and rosuvastatin concentration after the once-daily administration of the test sample of the present invention are summarized in Table 8. Additionally, the values of fenofibric acid concentration and rosuvastatin concentration after the once-daily administration of the reference sample are also shown in Table 8. Further, the plasma concentration-time profile after the once-daily administration of the test sample of the invention is shown for fenofibric acid and rosuvastatin respectively in Fig. 1 and Fig. 2.

**Table 8**

| Time points (h) | Mean plasma concentration of fenofibric acid after once-daily intake of the test sample (µg/mL) | Mean plasma concentration of rosuvastatin after once-daily intake of the test sample (ng/mL) | Mean plasma concentration of fenofibric acid after once-daily intake of SECALIP SUPRA^{®} 160 mg tablet (µg/mL) | Mean plasma concentration of rosuvastatin after once-daily intake of CRESTOR^{®} 20 mg tablet (ng/mL) |
|---|---|---|---|---|
| 0.000 | 0.000 | 0.000 | 0.000 | 0.000 |
| 0.333 | 0.000 | 1.790 | 0.000 | 0.875 |
| 0.667 | 0.136 | 2.905 | 0.137 | 2.322 |
| 1.000 | 0.652 | 5.094 | 0.676 | 4.007 |
| 1.333 | 1.610 | 7.293 | 1.372 | 5.489 |
| 1.667 | 2.610 | 9.255 | 2.190 | 7.357 |
| 2.000 | 3.464 | 10.586 | 3.161 | 8.765 |
| 2.333 | 4.251 | 10.992 | 3.928 | 9.872 |
| 2.667 | 4.912 | 11.982 | 4.557 | 11.166 |
| 3.000 | 5.474 | 12.600 | 5.452 | 13.310 |
| 3.333 | 5.882 | 12.850 | 6.264 | 14.163 |
| 3.667 | 6.172 | 13.200 | 6.688 | 14.114 |
| 4.000 | 6.551 | 13.429 | 7.424 | 14.802 |
| 4.500 | 7.461 | 13.938 | 8.320 | 16.315 |
| 5.000 | 8.176 | 14.148 | 9.032 | 16.061 |
| 5.500 | 9.302 | 16.721 | 10.202 | 18.177 |
| 6.000 | 8.812 | 13.307 | 9.335 | 13.704 |
| 8.000 | 6.765 | 6.612 | 7.267 | 7.121 |
| 10.000 | 5.686 | 4.490 | 6.264 | 4.771 |
| 12.000 | 5.158 | 3.382 | 5.608 | 3.624 |
| 16.000 | 3.536 | 1.769 | 3.814 | 1.988 |
| 20.000 | 2.866 | 1.257 | 3.078 | 1.398 |
| 24.000 | 2.530 | 0.950 | 2.644 | 1.008 |

Furthermore, the main pharmacokinetic parameters exhibited after the once-daily administration of the fixed dose combination according to the present invention, in the form of rosuvastatin and fenofibrate bilayer tablet of Example 4c and the once-daily administration of the reference sample products (SECALIP SUPRA^{®} 160 mg tablet and CRESTOR^{®} 20 mg tablet) were measured by means of LC-MS/MS method. Particularly, the values of the Cmax, Tmax, AUC, t½, (half life) and λz (elimination rate constant) as well as the standard deviation (SD) of the test sample and the reference sample were summarized in Table 9.

**Table 9.**

| **Pharmacok inetic parameters** | **Mean ± SD (untransformed data)** | | | |
|---|---|---|---|---|
| | **Fenofibric acid data from Test Sample** | **Rosuvastatin data from Test Sample** | **Fenofibric acid data from SECALIP SUPRA^{®} 160 mg tablet** | **Rosuvastatin data from CRESTOR^{®} 20 mg tablet** |
| **Tmax^{(a)}** | **5.509 (2.684-6.000) h** | **5.500 (1.667-6.000) h** | **5.500 (2.350-6.017) h** | **4.517 (1.684-5.533) h** |
| **Cmax** | **9.519 ± 1.8611 µg/mL** | **18.236 ± 5.9714 ng/mL** | **10.266 ± 1.9643 µg/mL** | **19.753 ± 7.8987 ng/mL** |
| **AUC0-t** | **172.468 ± 69.1113 µg.h/mL** | **135.959 ± 36.6152 ng·h/mL** | **181.135 ± 69.9180 µg.h/mL** | **142.116 ± 44.2050 ng·h/mL** |
| **AUC_{0-∞}** | **185.927 ± 80.2493 µg.h/mL** | **140.898 ± 36.3369 ng·h/mL** | **192.304 ± 77.2584 µg.h/mL** | **146.761 ± 44.7657 ng·h/mL** |
| **λ_{Z}** | **0.034 ± 0.0109 1/h** | **0.063 ± 0.0236 1/h** | **0.038 ± 0.0126 1/h** | **0.066 ± 0.0250 1/h** |
| **t½** | **22.363 ± 7.7019 h** | **13.389 ± 8.5600 h** | **20.161 ± 6.2812 h** | **12.600 ± 7.1307 h** |
| **AUC_%Ex trap_obs** | **6.178 ± 3.5430 %** | **3.701 ± 2.6124 %** | **5.339 ± 2.3882 %** | **3.289 ± 1.9971 %** |

| | | | | |
|---|---|---|---|---|
| (a) Tₘₐₓ is represented in median (min-max) value. | | | | |

The above pharmacokinetic parameters and the values of the mean plasma concentration of the fenofibric acid metabolite and rosuvastatin shown in Tables above surprisingly demonstrated that the once-daily administration of fixed dose combination according to the present invention, in the form of rosuvastatin and fenofibrate bilayer tablet of Example 4c, is bioequivalent to the total drug exposure obtained after once-daily administration of the concomitant intake of a 160 mg tablet of SECALIP SUPRA^{®} and a 20 mg tablet of CRESTOR^{®}. It means that the fixed dose combination according to the present invention has comparable bioavailability, because the rate and extent to which the fenofibric acid metabolite as well as the rosuvastatin is absorbed from the fixed dose combination according to the present invention and becomes available at the site of drug action is the same as the concomitant intake of the reference samples. Therefore, the once-daily administration of the fixed dose combination according to the present invention can be considered equivalent to once-daily administration of the immediate-release reference sample, i.e. the concomitant intake of a 160 mg tablet of SECALIP SUPRA ^{®} and a 20 mg tablet of CRESTOR^{®}.

In conclusion, the multilayer pharmaceutical composition of the invention comprising a fixed dose combination of rosuvastatin and fenofibrate, according to Example 4c, represents a relevant improvement over the corresponding monotherapies SECALIP SUPRA ^{®} and CRESTOR^{®}, as it provides the two active ingredients within one single tablet for once-daily treatment, thus reducing the patient pill burden. Thus, the fixed dose-combination is convenient to mixed dyslipidemia patients which currently need to take the two monotherapies separately, and thus covers an unmet medical need and provides an improvement to ensure treatment compliance and adherence.

## Claims

1. A multilayer pharmaceutical composition comprising a fixed dose combination of rosuvastatin or a pharmaceutically acceptable salt thereof and
fenofibrate or a pharmaceutically acceptable salt thereof, wherein the fenofibrate or the pharmaceutically acceptable salt thereof and the rosuvastatin or the pharmaceutically acceptable salt thereof are present in separate layers, namely a fenofibrate layer and a rosuvastatin layer, and wherein fenofibrate layer comprises micronized fenofibrate, and wherein fenofibrate and rosuvastatin are immediately released from the fenofibrate layer and the rosuvastatin layer, respectively.

2. The multilayer pharmaceutical composition according to claim 1, **characterized in that** the micronized fenofibrate has a volume median diameter D(v, 50) of more than 0.02 µm and less than or equal to 20 µm, preferably of more than 0.04 µm and less than or equal to 10 µm, more preferably of more than 0.05 µm and less than or equal to 7 µm, even more preferably of more than 0.05 µm and less than or equal to 5 µm, most preferably of more than 0.05 µm and less than or equal to 4 µm.

3. The multilayer pharmaceutical composition according to any of claims 1-2, **characterized in that** the micronized fenofibrate has a particle size distribution (PSD) in which
D(v,10) is more than 0.02 µm and less than or equal to 7 µm, preferably more than 0.04 µm and less than or equal to 5 µm, more preferably more than 0.04 µm and less than or equal to 4 µm, even more preferably more than 0.05 µm and less than or equal to 3 µm, even more preferably more than 0.05 µm and less than or equal to 2 µm; and/or
D(v,50) is more than 0.04 µm and less than or equal to 20 µm, preferably more than 0.08 µm and less than or equal to 10 µm, more preferably more than 0.1 µmand less than or equal to 7 µm, even more preferably more than 0.1 µm and less than or equal to 5 µm, even more preferably more than 0.1 µm and less than or equal to 4 µm; and/or
D(v,90) is more than 0.05 µm and less than or equal to 47 µm, preferably more than 0.1 µm and less than or equal to 25 µm, more preferably more than 0.2 µm and less than or equal to 18 µm, even more preferably more than 0.4 µm and lessthan or equal to 13 µm, even more preferably more than 0.5 µm and less than or equal to 10 µm; and
the width of the particle size distribution (PSD) expressed as span is comprised from 1.2 to 3, preferably from 1.3 to 2.5, more preferably from 1.6 to 2.3.

4. The multilayer pharmaceutical composition according to any of claims 1-3, **characterized in that** the fenofibrate is not in the form of a solid solution or solid dispersion.

5. The multilayer pharmaceutical composition according to any of claims 1-4, **characterized in that** the fenofibrate layer comprises less than 20% w/w or is free of one or more excipients or carriers with a melting point lower than 75°C, preferably lower than 80°C, more preferably lower than 90°C, even more preferably lower than 100°C.

6. The multilayer pharmaceutical composition according to claim 5, **characterized in that** the one or more excipients or carriers are selected from a group consisting of polyethylene glycols, polypropylene glycols, polyoxyethylenes, polyoxypropylenes, poloxamers, straight chain saturated hydrocarbons, sorbitan esters, paraffins, fats, oils and mixtures thereof.

7. The multilayer pharmaceutical composition according to any of claims 1 to 6, wherein rosuvastatin or pharmaceutically acceptable salt thereof is present in an amount from 5 mg to 20 mg, and fenofibrate or pharmaceutically acceptable salt is present in amount from 140 mg to 180 mg and rosuvastatin and fenofibrate are not mixed together.

8. The pharmaceutical composition according to claim 7, wherein the pharmaceutical composition is a bilayer tablet wherein said bilayer tablet comprises 5 to 20 mg rosuvastatin or pharmaceutically acceptable salt and 160 mg fenofibrate.

9. A process for preparing a pharmaceutical composition according to one of the preceding claims comprising the following steps:
a) preparing a lubricated fenofibrate layer blend by
i. preparing a fenofibrate blend by subjecting micronized fenofibrate, lactose monohydrate, microcrystalline cellulose, croscarmellose sodium, crospovidone, ferric oxide red and sodium lauryl sulphate to a granulation process;
ii. preparing a binder solution by dissolving povidone in purified water and adding iso propyl alcohol
iii. granulating the fenofibrate blend obtained in step i) and the binder solution obtained in step ii) and drying the granules in a suitable dryer;
iv. adding microcrystalline cellulose, crospovidone, croscarmellose sodium and colloidal anhydrous silica extragranularly to the granules obtained in step iii)
v. lubricating the dried granules with sodium steary fumarate;
b) preparing a lubricated rosuvastatin layer blend by
i. preparing a rosuvastatin blend mixing rosuvastatin calcium, lactose anhydrous, microcrystalline cellulose, crospovidone and light magnesium oxide in a blender;
ii. mixing sifted magnesium stearate with the rosuvastatin blend; and
c) compressing the lubricated fenofibrate layer blend and the lubricated rosuvastatin layer blend by a multilayer compression machine.

## Patentansprüche

1. Mehrschichtige pharmazeutische Zusammensetzung, umfassend eine fest dosierte Kombination von Rosuvastatin oder einem pharmazeutisch verträglichen Salz davon und
Fenofibrat oder einem pharmazeutisch verträglichen Salz davon, wobei das Fenofibrat oder das pharmazeutisch verträgliche Salz davon und das Rosuvastatin oder das pharmazeutisch verträgliche Salz davon in getrennten Schichten vorliegen, nämlich einer Fenofibratschicht und einer Rosuvastatinschicht, und wobei die Fenofibratschicht mikronisiertes Fenofibrat umfasst, und wobei Fenofibrat und Rosuvastatin unmittelbar aus der Fenofibratschicht bzw. der Rosuvastatinschicht freigesetzt werden.

2. Mehrschichtige pharmazeutische Zusammensetzung nach Anspruch 1, **dadurch gekennzeichnet, dass** das mikronisierte Fenofibrat einen mittleren Volumendurchmesser D(v, 50) von mehr als 0,02 µm und weniger als oder gleich 20 µm, bevorzugt von mehr als 0,04 µm und weniger als oder gleich 10 µm, stärker bevorzugt von mehr als 0,05 µm und weniger als oder gleich 7 µm, noch stärker bevorzugt von mehr als 0,05 µm und weniger als oder gleich 5 µm, am meisten bevorzugt von mehr als 0,05 µm und weniger als oder gleich 4 µm aufweist.

3. Mehrschichtige pharmazeutische Zusammensetzung nach einem der Ansprüche 1-2, **dadurch gekennzeichnet, dass** das mikronisierte Fenofibrat eine Partikelgrößenverteilung (PSD) aufweist, bei der
D(v,10) mehr als 0,02 µm und weniger als oder gleich 7 µm, bevorzugt mehr als 0,04 µm und weniger als oder gleich 5 µm, stärker bevorzugt mehr als 0,04 µm und weniger als oder gleich 4 µm, noch stärker bevorzugt mehr als 0,05 µm und weniger als oder gleich 3 µm, noch stärker bevorzugt mehr als 0,05 µm und weniger als oder gleich 2 µm beträgt; und/oder
D(v,50) mehr als 0,04 µm und weniger als oder gleich 20 µm, bevorzugt mehr als 0,08 µm und weniger als oder gleich 10 µm, stärker bevorzugt mehr als 0,1 µm und weniger als oder gleich 7 µm, noch stärker bevorzugt mehr als 0,1 µm und weniger als oder gleich 5 µm, noch stärker bevorzugt mehr als 0,1 µm und weniger als oder gleich 4 µm beträgt; und/oder
D(v,90) mehr als 0,05 µm und weniger als oder gleich 47 µm, bevorzugt mehr als 0,1 µm und weniger als oder gleich 25 µm, stärker bevorzugt mehr als 0,2 µm und weniger als oder gleich 18 µm, noch stärker bevorzugt mehr als 0,4 µm und weniger als oder gleich 13 µm, noch stärker bevorzugt mehr als 0,5 µm und weniger als oder gleich 10 µm beträgt; und
die Breite der Partikelgrößenverteilung (PSD), ausgedrückt als Spanne, von 1,2 bis 3, bevorzugt von 1,3 bis 2,5, noch stärker bevorzugt von 1,6 bis 2,3 reicht.

4. Mehrschichtige pharmazeutische Zusammensetzung nach einem der Ansprüche 1-3, **dadurch gekennzeichnet, dass** das Fenofibrat nicht in Form einer festen Lösung oder festen Dispersion vorliegt.

5. Mehrschichtige pharmazeutische Zusammensetzung nach einem der Ansprüche 1-4, **dadurch gekennzeichnet, dass** die Fenofibratschicht weniger als 20% w/w umfasst oder frei von einem oder mehreren Hilfsstoffen oder Trägern mit einem Schmelzpunkt unter 75°C, bevorzugt unter 80 °C, stärker bevorzugt unter 90 °C und noch stärker bevorzugt unter 100°C ist.

6. Mehrschichtige pharmazeutische Zusammensetzung nach Anspruch 5, **dadurch gekennzeichnet, dass** der eine oder die mehreren Hilfsstoffe oder Träger aus einer Gruppe ausgewählt sind, die aus Polyethylenglykolen, Polypropylenglykolen, Polyoxyethylenen, Polyoxypropylenen, Poloxameren, geradkettigen gesättigten Kohlenwasserstoffen, Sorbitanestern, Paraffinen, Fetten, Ölen und Mischungen davon besteht.

7. Mehrschichtige pharmazeutische Zusammensetzung nach einem der Ansprüche 1 bis 6, wobei Rosuvastatin oder ein pharmazeutisch verträgliches Salz davon in einer Menge von 5 mg bis 20 mg und Fenofibrat oder ein pharmazeutisch verträgliches Salz davon in einer Menge von 140 mg bis 180 mg vorhanden ist und Rosuvastatin und Fenofibrat nicht miteinander vermischt sind.

8. Pharmazeutische Zusammensetzung nach Anspruch 7, wobei die pharmazeutische Zusammensetzung eine zweischichtige Tablette ist, wobei die zweischichtige Tablette 5 bis 20 mg Rosuvastatin oder ein pharmazeutisch verträgliches Salz und 160 mg Fenofibrat enthält.

9. Verfahren zur Herstellung einer pharmazeutischen Zusammensetzung nach einem der vorhergehenden Ansprüche, umfassend die folgenden Schritte:
a) Herstellen einer geschmierten Fenofibratschicht-Mischung durch
i. Herstellen einer Fenofibratmischung, indem mikronisiertes Fenofibrat, Lactose-Monohydrat, mikrokristalline Cellulose, Croscarmellose-Natrium, Crospovidon, Eisenoxidrot und Natriumlaurylsulfat einem Granulierungsverfahren unterzogen werden;
ii. Herstellen einer Bindemittellösung durch Auflösen von Povidon in gereinigtem Wasser und Zugeben von Isopropylalkohol
iii. Granulieren der in Schritt i) erhaltenen Fenofibratmischung und der in Schritt ii) erhaltenen Bindemittellösung und Trocknen des Granulats in einem geeigneten Trockner;
iv. extragranulares Zugeben von mikrokristalliner Cellulose, Crospovidon, Croscarmellose-Natrium und kolloidalem wasserfreiem Siliciumdioxid zu dem in Schritt iii) erhaltenen Granulat
v. Schmieren des getrockneten Granulats mit Natriumstearylfumarat;
b) Herstellen einer geschmierten Rosuvastatinschicht-Mischung durch
i. Herstellen einer Rosuvastatin-Mischung durch Mischen von Rosuvastatin-Calcium, wasserfreier Laktose, mikrokristalliner Cellulose, Crospovidon und leichtem Magnesiumoxid in einem Mixer;
ii. Mischen von gesiebtem Magnesiumstearat mit der Rosuvastatinmischung; und
c) Komprimieren der geschmierten Fenofibratschicht-Mischung und der geschmierten Rosuvastatinschicht-Mischung durch eine Mehrschicht-Kompressionsmaschine.

## Revendications

1. Composition pharmaceutique multicouche comportant une combinaison de doses fixe de rosuvastatine ou d'un sel pharmaceutiquement acceptable de celle-ci et
d'un fénofibrate ou d'un sel pharmaceutiquement acceptable de celui-ci, dans laquelle le fénofibrate ou le sel pharmaceutiquement acceptable de celui-ci et la rosuvastatine ou le sel pharmaceutiquement acceptable de celle-ci sont présents dans des couches séparées, à savoir une couche de fénofibrate et une couche de rosuvastatine, et dans laquelle la couche de fénofibrate comporte du fénofibrate micronisé, et dans laquelle le fénofibrate et la rosuvastatine sont immédiatement libérés à partir de la couche de fénofibrate et de la couche de rosuvastatine, respectivement.

2. Composition pharmaceutique multicouche selon la revendication 1, **caractérisée en ce que** le fénofibrate micronisé a un diamètre volumique médian D(v, 50) supérieur à 0,02 µm et inférieur ou égal à 20 µm, de manière préférée supérieur à 0,04 µm et inférieur ou égal à 10 µm, de manière plus préférée supérieur à 0,05 µm et inférieur ou égal à 7 µm, de manière encore plus préférée supérieur à 0,05 µm et inférieur ou égal à 5 µm, de manière la plus préférée supérieur à 0,05 µm et inférieur ou égal à 4 µm.

3. Composition pharmaceutique multicouche selon l'une quelconque des revendications 1 à 2, **caractérisée en ce que** le fénofibrate micronisé a une distribution granulométrique (PSD) dans laquelle
D(v,10) est supérieur à 0,02 µm et inférieur ou égal à 7 µm, de manière préférée supérieur à 0,04 µm et inférieur ou égal à 5 µm, de manière plus préférée supérieur à 0,04 µm et inférieur ou égal à 4 µm, de manière encore plus préférée supérieur à 0,05 µm et inférieur ou égal à 3 µm, de manière encore plus préférée supérieur à 0,05 µm et inférieur ou égal à 2 µm ; et/ou
D(v,50) est supérieur à 0,04 µm et inférieur ou égal à 20 µm, de manière préférée supérieur à 0,08 µm et inférieur ou égal à 10 µm, de manière plus préférée supérieur à 0,1 µm et inférieur ou égal à 7 µm, de manière encore plus préférée supérieur à 0,1 µm et inférieur ou égal à 5 µm, de manière encore plus préférée supérieur à 0,1 µm et inférieur ou égal à 4 µm ; et/ou
D(v,90) est supérieur à 0,05 µm et inférieur ou égal à 47 µm, de manière préférée supérieur à 0,1 µm et inférieur ou égal à 25 µm, de manière plus préférée supérieur à 0,2 µm et inférieur ou égal à 18 µm, de manière encore plus préférée supérieur à 0,4 µm et inférieur ou égal à 13 µm, de manière encore plus préférée supérieur à 0,5 µm et inférieur ou égal à 10 µm ; et
la largeur de la distribution granulométrique (PSD) exprimée sous forme d'intervalle est comprise entre 1,2 et 3, de manière préférée entre 1,3 et 2,5, de manière plus préférée entre 1,6 et 2,3

4. Composition pharmaceutique multicouche selon l'une quelconque des revendications 1 à 3, **caractérisée en ce que** le fénofibrate ne se présente pas sous la forme d'une solution solide ou d'une dispersion solide.

5. Composition pharmaceutique multicouche selon l'une quelconque des revendications 1 à 4, **caractérisée en ce que** la couche de fénofibrate comporte moins de 20 % p/p ou est exempte d'un ou plusieurs excipients ou vecteurs avec un point de fusion inférieur à 75 °C, de manière préférée inférieur à 80 °C, de manière plus préférée inférieur à 90 °C, de manière encore plus préférée inférieur à 100 °C.

6. Composition pharmaceutique multicouche selon la revendication 5, **caractérisée en ce que** le ou les excipients ou vecteurs sont choisis parmi un groupe constitué de polyéthylène glycols, de polypropylène glycols, de polyoxyéthylènes, de polyoxypropylènes, de poloxamères, d'hydrocarbures saturés à chaîne droite, d'esters de sorbitan, de paraffines, de graisses, d'huiles et de mélanges de ceux-ci.

7. Composition pharmaceutique multicouche selon l'une quelconque des revendications 1 à 6, dans laquelle la rosuvastatine ou un sel pharmaceutiquement acceptable de celle-ci est présent en une quantité de 5 mg à 20 mg, et le fénofibrate ou un sel pharmaceutiquement acceptable de celui-ci est présent en une quantité de 140 mg à 180 mg et la rosuvastatine et le fénofibrate ne sont pas mélangés ensemble.

8. Composition pharmaceutique selon la revendication 7, dans laquelle la composition pharmaceutique est un comprimé bicouche, dans laquelle ledit comprimé bicouche comporte 5 à 20 mg de rosuvastatine ou d'un sel pharmaceutiquement acceptable et 160 mg de fénofibrate.

9. Procédé pour préparer une composition pharmaceutique selon l'une des revendications précédentes, comportant les étapes suivantes consistant à :
a) préparer un mélange de couches de fénofibrate lubrifié par
i. préparation d'un mélange de fénofibrates en soumettant du fénofibrate micronisé, du monohydrate de lactose, de la cellulose microcristalline, de la croscarmellose sodique, de la crospovidone, de l'oxyde ferrique rouge et du sulfate de sodium lauryl à un procédé de granulation ;
ii. préparation d'une solution de liant en dissolvant de la povidone dans de l'eau purifiée et en ajoutant de l'alcool isopropylique
iii. granulation du mélange de fénofibrates obtenu à l'étape i) et la solution de liant obtenue à l'étape ii) et séchage des granules dans un sécheur adapté ;
iv. ajout de la cellulose microcristalline, de la crospovidone, de la croscarmellose sodique et de la silice anhydre colloïdale de manière extragranulaire aux granules obtenus à l'étape iii)
v. lubrification des granules séchés avec du stéaryl fumarate de sodium ;
b) préparer un mélange de couches de rosuvastatine lubrifié par
i. préparation d'un mélange de rosuvastatine en mélangeant du calcium de rosuvastatine, du lactose anhydre, de la cellulose microcristalline, de la crospovidone et un oxyde de magnésium léger dans un mélangeur ;
ii. mélange du stéarate de magnésium criblé avec le mélange de rosuvastatine ; et
c) comprimer le mélange de couches de fénofibrate lubrifié et le mélange de couches de rosuvastatine lubrifié par une machine de compression multicouche.
